(19) Europäisches Patentamt / European Patent Office / Office européen des brevets

(11) **EP 4 023 149 B1**

(12) **EUROPEAN PATENT SPECIFICATION**

(45) Date of publication and mention
of the grant of the patent:
**22.10.2025   Bulletin 2025/43**

(21) Application number: **20871855.1**

(22) Date of filing: **02.09.2020**

(51) International Patent Classification (IPC):
*A61B 5/0225* *(2006.01)*    *A61B 5/022* *(2006.01)*
*A61B 5/021* *(2006.01)*    *A61B 5/02* *(2006.01)*
*A61B 5/00* *(2006.01)*

(52) Cooperative Patent Classification (CPC):
**A61B 5/02116; A61B 5/681; A61B 5/6824;**
**A61B 5/6843;** A61B 5/02225; A61B 5/7239

(86) International application number:
**PCT/CN2020/113102**

(87) International publication number:
**WO 2021/063150 (08.04.2021 Gazette 2021/14)**

(54) **BLOOD PRESSURE MEASUREMENT METHOD AND ELECTRONIC DEVICE**

BLUTDRUCKMESSVERFAHREN UND ELEKTRONISCHE VORRICHTUNG

PROCÉDÉ DE MESURE DE LA PRESSION ARTÉRIELLE ET DISPOSITIF ÉLECTRONIQUE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **30.09.2019   CN 201910940521**

(43) Date of publication of application:
**06.07.2022   Bulletin 2022/27**

(73) Proprietor: **Huawei Technologies Co., Ltd.**
**Longgang District**
**Shenzhen,**
**Guangdong 518129 (CN)**

(72) Inventor: **KUANG, Yunsheng**
**Shenzhen, Guangdong 518129 (CN)**

(74) Representative: **Pfenning, Meinig & Partner mbB**
**Patent- und Rechtsanwälte**
**Theresienhöhe 11a**
**80339 München (DE)**

(56) References cited:
CN-A- 1 689 508         CN-A- 102 307 519
CN-A- 109 984 736       CN-A- 109 984 736
CN-A- 110 123 294       CN-A- 110 151 155
US-A1- 2009 118 628     US-A1- 2011 295 130
US-B1- 6 336 901

# Description

## TECHNICAL FIELD

This application relates to the field of electronic devices, and in particular, to a blood pressure measurement method and an electronic device.

## BACKGROUND

Currently, an electronic sphygmomanometer has become a mainstream product of a sphygmomanometer. Most of mainstream electronic sphygmomanometers in the market are upper arm electronic sphygmomanometers. Although the electronic sphygmomanometer can meet a requirement of a user for blood pressure measurement, the electronic sphygmomanometer cannot be worn for a long time in terms of a size and weight of the device. The electronic sphygmomanometer cannot meet some requirements for long-term blood pressure measurement (for example, nighttime blood pressure measurement, real-time blood pressure tracking and detection, and blood pressure feedback control). These requirements are just important means to effectively prevent sudden diseases such as hypertension and a cerebral stroke, and are rigid requirements of the user, especially a hypertensive patient.

Because a wrist sphygmomanometer (for example, a blood pressure watch or a blood pressure wristband) has a smaller size and lighter weight, the wrist sphygmomanometer can be worn by the user for a long time, and meets a requirement for long-term and real-time blood pressure detection. A basic principle of measuring blood pressure by the wrist sphygmomanometer is as follows: When the wrist sphygmomanometer is worn on a wrist of the user, an airbag included in the wrist sphygmomanometer is closely attached to the radial artery of the user, and therefore it may be considered that a pressure value of the airbag is approximately equal to a pressure value of the radial artery. The wrist sphygmomanometer can determine a blood pressure value of the user by detecting the pressure value of the airbag. The blood pressure value may include systolic blood pressure (systolic blood pressure, SBP) and diastolic blood pressure (diastolic blood pressure, DBP).

During blood pressure measurement, wearing tightness of the wrist sphygmomanometer significantly affects blood pressure measurement accuracy. For example, if the sphygmomanometer is worn too loosely, the pressure value of the airbag is greater than the pressure value of the radial artery, and therefore a measured blood pressure is higher. For another example, if the sphygmomanometer is worn too tightly, the pressure value of the airbag is less than the pressure value of the radial artery, and therefore a measured blood pressure value is lower.

US 2011/295130 A1 discloses a blood pressure measurement device includes a wrapping strength detecting portion for detecting a wrapping strength of a cuff with respect to a measurement site. The wrapping strength detecting portion detects the wrapping strength of the cuff prior to calculation of a blood pressure value by a blood pressure calculation unit.

CN 109984736 A discloses a blood pressure measuring device, which includes a device body, a wrist strap and a pulse sensor. The device body is connected to the wrist strap and comprises a shell, a processor, a driving component and a pressure sensor. Other relevant prior art is disclosed in US 6 336 901 B1.

## SUMMARY

The invention is set out in the appended set of claims. Embodiments of this application provide a blood pressure measurement method and an electronic device, to resolve at least a problem of low blood pressure measurement accuracy caused by different wearing tightness.

To achieve the foregoing objective, the following technical solutions are used in the embodiments of this application.

According to a first aspect, an embodiment of this application provides a blood pressure measurement method. The method may be applied to an electronic device, the electronic device can be worn on a wrist of a user by using a wristband of the electronic device, and the electronic device includes an airbag and an air pump. The method may include: When the electronic device is worn on the wrist of the user, the electronic device controls the air pump to inflate and pressurize the airbag. The electronic device obtains a pressure value of the airbag in a process of controlling the air pump to inflate and pressurize the airbag. The electronic device obtains a first pressure signal, where the first pressure signal is a signal indicating that the pressure value of the airbag changes with time in a process in which the air pump starts to inflate and pressurize the airbag until the pressure value of the airbag is equal to a preset pressure value. The electronic device determines, based on the first pressure signal, tightness of wearing the wristband by the user. The electronic device corrects, based on the tightness, a blood pressure measurement value determined based on a second pressure signal, to determine a blood pressure value of the user, where the second pressure signal is a signal indicating that the pressure value of the airbag changes with time after the pressure value of the airbag exceeds the preset pressure value.

In this way, the electronic device determines, by analyzing an original signal (that is, the first pressure signal) in an initial pressurization phase, the tightness of wearing wristband by the user, and corrects the blood pressure measurement value based on the tightness, that is, adds a difference between blood pressure measurement results caused by different wearing tightness to the blood pressure measurement value, to improve blood pressure measurement accuracy.

In a possible design, that the electronic device determines, based on the first pressure signal, tightness of wearing the wristband by the user includes: The electronic device determines a pressurization slope of the first pressure signal, where the pressurization slope is a slope of a curve of the first pressure signal, and the pressurization slope is used to indicate the tightness of wearing the wristband by the user. That the electronic device corrects, based on the tightness, a blood pressure measurement value determined based on a second pressure signal, to determine a blood pressure value of the user includes: The electronic device corrects the blood pressure measurement value based on the pressurization slope of the first pressure signal, to determine the blood pressure value. In this way, the electronic device quantizes the wearing tightness based on the slope of the curve of the original signal (that is, the first pressure signal) in the initial pressurization phase, and provides corresponding compensation for the blood pressure measurement value based on the slope, to obtain a more accurate blood pressure value.

In a possible design, that the electronic device corrects the blood pressure measurement value based on the pressurization slope of the first pressure signal, to determine the blood pressure value includes: The electronic device determines a compensation value based on the pressurization slope of the first pressure signal and a mapping relationship, where the mapping relationship includes a mapping relationship between the pressurization slope of the first pressure signal and the compensation value. The electronic device corrects the blood pressure measurement value by using the compensation value, to determine the blood pressure value. In this way, the electronic device may determine, based on the pressurization slope and the mapping relationship, a value that needs to be used to compensate for the blood pressure measurement value with current wearing tightness, so that the electronic device corrects the blood pressure measurement value based on the value that needs to be used to compensate for the blood pressure measurement value.

In a possible design, that the electronic device corrects the blood pressure measurement value based on the pressurization slope of the first pressure signal, to determine the blood pressure value includes: When determining that the pressurization slope is greater than a first slope and less than a second slope, the electronic device corrects the blood pressure measurement value based on the pressurization slope of the first pressure signal, to determine the blood pressure value. In this way, the electronic device may determine that a pressurization slope in current blood pressure measurement falls within a preset range (for example, between the first slope and the second slope) in which the blood pressure measurement value can be accurately compensated for, so that the electronic device can more accurately obtain the blood pressure value of the user by modifying the blood pressure measurement value based on the pressurization slope.

In a possible design, the method further includes: When determining that the pressurization slope is less than the first slope, the electronic device controls the air pump to stop inflating and pressurizing the airbag, and prompts the user to tighten the wristband. When the electronic device determines that the pressurization slope is greater than the second slope, the electronic device controls the air pump to stop inflating and pressurizing the airbag, and prompts the user to loosen the wristband. In this way, the electronic device may determine that in a current wearing state in which the wristband is worn too loosely or too tightly, an accurate blood pressure value cannot be obtained even if a measurement result is compensated for. In this case, the electronic device stops the measurement, and prompts the user to tighten or loosen the wristband, so that the wearing tightness can be adjusted to a range in which blood pressure can be accurately measured.

That the electronic device determines, based on the first pressure signal, tightness of wearing the wristband by the user includes: The electronic device determines, based on the first pressure signal, a pressurization time period from a time point at which the airbag starts to be pressurized to a time point at which the pressure value of the airbag is equal to the preset pressure value, where the pressurization time period is used to indicate the tightness of wearing the wristband by the user. That the electronic device corrects, based on the tightness, a blood pressure measurement value determined based on a second pressure signal, to determine a blood pressure value of the user includes: The electronic device corrects the blood pressure measurement value based on the pressurization time period, to determine the blood pressure value. In this way, the electronic device quantizes the wearing tightness based on the pressurization time period of the curve of the first pressure signal, that is, a time period from a time point at which the pressure value of the airbag starts to be increased to a time point at which the pressure value of the airbag reaches the preset pressure value, and provides corresponding compensation for the blood pressure measurement value based on the time period, to obtain a more accurate blood pressure value.

In a possible design, that the electronic device corrects the blood pressure measurement value based on the pressurization time period, to determine the blood pressure value includes: The electronic device determines a compensation value based on the pressurization time period and a mapping relationship, where the mapping relationship includes a mapping relationship between the pressurization time period and the compensation value. The electronic device corrects the blood pressure measurement value by using the compensation value, to determine the blood pressure value. In this way, the electronic device may determine, based on the pressurization time period and the mapping relationship, a value that needs to be used to compensate for the blood pressure measurement value with current wearing tightness, so that the electronic device corrects the blood pressure measurement value based on the value that needs to be used to compensate for the blood pressure measurement value.

In a possible design, that the electronic device corrects the blood pressure measurement value based on the pressurization time period, to determine the blood pressure value includes: When determining that the pressurization time period is greater than a first time period and less than a second time period, the electronic device corrects the blood pressure measurement value based on the pressurization time period, to determine the blood pressure value. In this way, the electronic device can determine that in a current wearing state, accurate compensation can be provided for the blood pressure measurement value based on the wearing tightness (for example, the pressurization time period).

In a possible design, the method further includes: When determining that the pressurization time period is less than the first time period, the electronic device controls the air pump to stop inflating and pressurizing the airbag, and prompts the user to tighten the wristband. When determining that the pressurization time period is greater than the second time period, the electronic device controls the air pump to stop inflating and pressurizing the airbag, and prompts the user to loosen the wristband. In this way, the electronic device may determine that in a current wearing state in which the wristband is worn too loosely or too tightly, an accurate blood pressure value cannot be obtained even if a measurement result is compensated for. In this case, the electronic device stops the measurement, and prompts the user to tighten or loosen the wristband, so that the wearing tightness can be adjusted to a range in which blood pressure can be accurately measured.

According to a second aspect, an embodiment of this application provides an electronic device. The electronic device can be worn on a wrist of a user by using a wristband of the electronic device, and the electronic device includes a microcontroller unit MCU, a pressure sensor, an airbag, and an air pump. The MCU is configured to: when the electronic device is worn on the wrist of the user, control the air pump to inflate and pressurize the airbag. The pressure sensor is configured to obtain a pressure value of the airbag in a process of controlling the air pump to inflate and pressurize the airbag. The MCU is further configured to obtain a first pressure signal, where the first pressure signal is a signal indicating that the pressure value of the airbag changes with time in a process in which the air pump starts to inflate and pressurize the airbag until the pressure value of the airbag is equal to a preset pressure value. The MCU is further configured to determine, based on the first pressure signal, tightness of wearing the wristband by the user. The MCU is further configured to correct, based on the tightness, a blood pressure measurement value determined based on a second pressure signal, to determine a blood pressure value of the user, where the second pressure signal is a signal indicating that the pressure value of the airbag changes with time after the pressure value of the airbag exceeds the preset pressure value.

In a possible design, that the MCU is further configured to determine, based on the first pressure signal, tightness of wearing the wristband by the user includes: The MCU is configured to determine a pressurization slope of the first pressure signal, where the pressurization slope is a slope of a curve of the first pressure signal, and the pressurization slope is used to indicate the tightness of wearing the wristband by the user. That the MCU is further configured to correct, based on the tightness, a blood pressure measurement value determined based on a second pressure signal, to determine a blood pressure value of the user includes: The MCU is configured to correct the blood pressure measurement value based on the pressurization slope of the first pressure signal, to determine the blood pressure value.

In a possible design, that the MCU is configured to correct the blood pressure measurement value based on the pressurization slope of the first pressure signal, to determine the blood pressure value includes: The MCU is configured to determine a compensation value based on the pressurization slope of the first pressure signal and a mapping relationship, where the mapping relationship includes a mapping relationship between the pressurization slope of the first pressure signal and the compensation value. The MCU is further configured to correct the blood pressure measurement value by using the compensation value, to determine the blood pressure value.

In a possible design, that the MCU is configured to correct the blood pressure measurement value based on the pressurization slope of the first pressure signal, to determine the blood pressure value includes: When determining that the pressurization slope is greater than a first slope and less than a second slope, the MCU corrects the blood pressure measurement value based on the pressurization slope of the first pressure signal, to determine the blood pressure value.

In a possible design, the MCU is further configured to: when determining that the pressurization slope is less than the first slope, control the air pump to stop inflating and pressurizing the airbag, and prompt the user to tighten the wristband. The MCU is further configured to: when determining that the pressurization slope is greater than the second slope, control the air pump to stop inflating and pressurizing the airbag, and prompt the user to loosen the wristband.

That the MCU is further configured to determine, based on the first pressure signal, tightness of wearing the wristband by the user includes: The MCU is configured to determine, based on the first pressure signal, a pressurization time period from a time point at which the airbag starts to be pressurized to a time point at which the pressure value of the airbag is equal to the preset pressure value, where the pressurization time period is used to indicate the tightness of wearing the wristband by the user. That the MCU is further configured to correct, based on the tightness, a blood pressure measurement value determined based on a second pressure signal, to determine a blood pressure value of the user includes: The MCU is configured to correct the blood pressure measurement value based on the pressurization time period, to determine the blood pressure value.

In a possible design, that the MCU is configured to correct the blood pressure measurement value based on the pressurization time period, to determine the blood pressure value includes: The MCU is configured to determine a compensation value based on the pressurization time period and a mapping relationship, where the mapping relationship includes a mapping relationship between the pressurization time period and the compensation value. The MCU is further configured to correct the blood pressure measurement value by using the compensation value, to determine the blood pressure value.

In a possible design, that the MCU is configured to correct the blood pressure measurement value based on the pressurization time period, to determine the blood pressure value includes: When determining that the pressurization time period is greater than a first time period and less than a second time period, the MCU corrects the blood pressure measurement value based on the pressurization time period, to determine the blood pressure value.

In a possible design, the MCU is further configured to: when determining that the pressurization time period is less than the first time period, control the air pump to stop inflating and pressurizing the airbag, and prompt the user to tighten the wristband. The MCU is further configured to: when determining that the pressurization time period is greater than the second time period, control the air pump to stop inflating and pressurizing the airbag, and prompt the user to loosen the wristband.

It may be understood that the electronic device according to the second aspect is configured to perform the corresponding method provided above. Therefore, for beneficial effects that can be achieved by the electronic device refer to beneficial effects in the corresponding method provided above. Details are not described again herein.

## BRIEF DESCRIPTION OF DRAWINGS

FIG. 1 is a schematic diagram of a blood pressure measurement method according to the conventional technology;
FIG. 2 is a schematic composition diagram of an electronic device according to an embodiment of this application;
FIG. 3(a) and FIG. 3(b) are a schematic diagram of a product form of an electronic device according to an embodiment of this application;
FIG. 4(a), FIG. 4(b), and FIG. 4(c) are a schematic diagram of comparison between waveforms of an original signal in an initial pressurization phase with different wearing tightness;
FIG. 5 is a schematic flowchart of a blood pressure measurement method according to an embodiment of this application;
FIG. 6(a) and FIG. 6(b) are a schematic diagram of a method for triggering blood pressure measurement according to an embodiment of this application;
FIG. 7 is a schematic diagram of a method for calculating a pressurization slope according to an embodiment of this application;
FIG. 8(a) and FIG. 8(b) are a schematic diagram of prompting a user to adjust wearing tightness according to an embodiment of this application;
FIG. 9 is a schematic diagram of a mapping relationship between an SBP compensation value and a pressurization slope according to an embodiment of this application;
FIG. 10 is a schematic flowchart of another blood pressure measurement method according to an embodiment of this application;
FIG. 11 is a schematic diagram of a mapping relationship between an SBP compensation value and a pressurization time period according to an embodiment of this application; and
FIG. 12 is a schematic diagram of logical composition of an electronic device according to an embodiment of this application.

## DESCRIPTION OF EMBODIMENTS

A wrist sphygmomanometer can meet a requirement for measuring blood pressure by a user in real time, and therefore is gradually widely used. The wrist sphygmomanometer mainly includes an airbag, an air pump, and a pressure sensor. During blood pressure measurement, the wrist sphygmomanometer controls the air pump to inflate the airbag. Therefore, the airbag is pressurized and inflated, and compresses the radial artery of a wrist. Because the airbag is closely attached to the radial artery of the user, the wrist sphygmomanometer may detect a pressure value near the radial artery by monitoring pressure of the airbag by using the pressure sensor, and further measure blood pressure of the user.

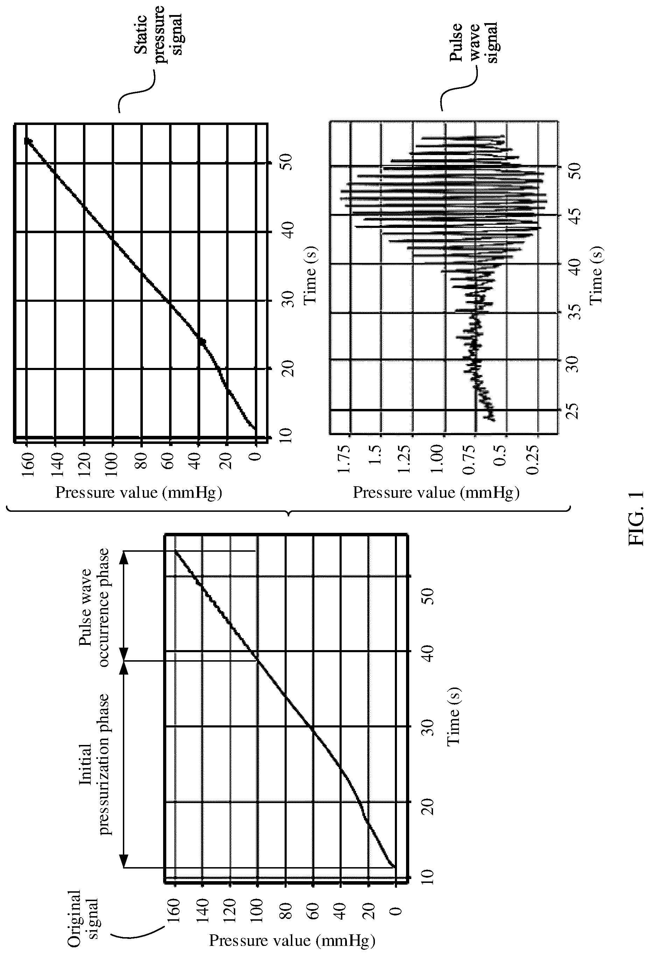

For example, in a process in which the airbag is inflated and pressurized, the pressure sensor in the wrist sphygmomanometer may detect real-time pressure of the airbag in real time. The wrist sphygmomanometer may generate, based on the real-time pressure, an original signal shown in FIG. 1. As shown in FIG. 1, in an initial pressurization phase, a pressure value of the airbag is still different from a pressure value of the radial artery of the user, and therefore smoothly rises in the original signal. After the initial pressurization phase ends, the pressure value of the airbag is close to the pressure value of the radial artery of the user, and therefore a pulse wave signal with subtle but continuous fluctuation occurs in the original signal (a pulse wave occurrence phase shown in FIG. 1). The wrist sphygmomanometer may obtain a linearly varying static pressure signal and a fluctuating pulse wave signal with characteristic information by analyzing the original signal, and further determine blood pressure of the user based on the static pressure signal and the characteristic information of the pulse wave signal.

It should be noted that, the foregoing process is performed on the premise that air pressure in the airbag is equal to pressure for compressing the radial artery, and a pulse wave signal at the radial artery is extracted by obtaining a change of the air pressure in the airbag, to further determine blood pressure of the user. However, in practice, when the wrist sphygmomanometer is worn with different tightness, the air pressure in the airbag is not necessarily the same as a value of the pressure for compressing the radial artery.

For example, if the wrist sphygmomanometer is worn moderately, because the airbag is closely attached to the wrist but is not under force, pressure of inflation of the airbag is basically completely transmitted to pressure for compressing the wrist. In this case, the pressure value of the airbag is approximately the same as the pressure value of the radial artery. The pulse wave signal obtained by the wrist sphygmomanometer through measurement can accurately reflect the blood pressure of the user, so that a blood pressure measurement result is accurate.

If the wrist sphygmomanometer is worn loosely, because the airbag is not attached to the wrist, in a pressurization process, the airbag is attached to skin of the wrist only a part of the airbag is inflated, and the pressure is transmitted to the pressure for compressing the radial artery. Specific pressure is required for the inflation of the airbag. Consequently, the pressure of the airbag is greater than the pressure for compressing the radial artery. In this way, the entire pulse wave signal obtained by the wrist sphygmomanometer is offset along a high pressure direction. Consequently, the measured blood pressure is greater than actual blood pressure. For example, it is proved by experiments that if tightness of wearing a wristband is 5 mm less than normal wearing tightness, an average value of peak pressure of a pulse wave changes from 113.4 mmHg to 135.9 mmHg, and is offset by more than 20 mmHg. To be specific, if the wristband is worn too loosely, significant deviations of a blood pressure value calculated based on the peak pressure of the pulse wave may occur.

If the wrist sphygmomanometer is worn too tightly, before pressurization measurement is started, the airbag bound through the wristband already exerts pressure on the wrist, and compresses the radial artery. In this way, in a pressurization process, the pressure of the airbag needs to be less than the pressure for compressing the radial artery, and the entire pulse wave signal is offset along a low pressure direction. Consequently, the measured blood pressure is less than actual blood pressure.

To be specific, the wearing tightness of the wrist sphygmomanometer significantly affects blood pressure measurement accuracy. A hard support with an elliptical-like ring structure may be disposed to wear the wrist sphygmomanometer in the wristband on the wrist of the user, so as to reduce impact of the wearing tightness on the measurement result. A notch is disposed in the middle of the wristband with the hard support, and the airbag is stuck on the hard support. When the blood pressure watch is worn, the hard support is stuck at the wrist. Therefore, the airbag is closely attached to the wrist but does not exert pressure on the wrist, to ensure that the pressure value of the airbag is the same as the pressure value of the radial artery. However, due to individual differences in wrist sizes, a specification size of the hard support cannot be well applied to different users. Therefore, it cannot be ensured that the airbag is closely attached to the wrist without exerting pressure on the wrist, and significant wrist discomfort also occurs when the user wears the wrist sphygmomanometer.

To resolve the foregoing problem, an embodiment of this application provides a blood pressure measurement method. The method may be applied to an electronic device. The electronic device may measure blood pressure by wearing a wristband on a wrist of a user. The electronic device may determine wearing tightness based on a collected original signal in an initial pressurization phase, and correct a measured blood pressure value based on the tightness. In this way, at least a problem of low blood pressure measurement accuracy caused by different wearing tightness can be resolved.

The following describes implementations of the embodiments of this application in detail with reference to the accompanying drawings.

For example, the electronic device in the embodiments of this application may be a device that has a blood pressure measurement function such as a mobile phone, a tablet computer, a desktop computer, a laptop computer, a handheld computer, a notebook computer, an ultra-mobile personal computer (ultra-mobile personal computer, UMPC), a netbook, a cellular phone, a personal digital assistant (personal digital assistant, PDA), an augmented reality (augmented reality, AR)/virtual reality (virtual reality, VR) device, or a wearable device (for example, a smart watch or a wrist sphygmomanometer (for example, a blood pressure watch or a blood pressure wristband)). A specific form of the device is not specifically limited in this embodiment of this application.

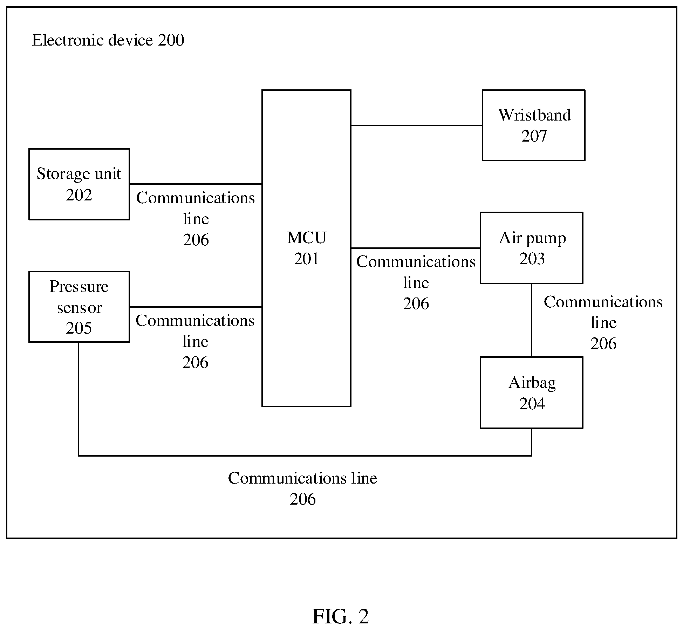

FIG. 2 is a schematic composition diagram of an electronic device according to an embodiment of this application.

As shown in FIG. 2, the electronic device 200 may include a microcontroller unit (Microcontroller Unit, MCU) 201, a storage unit 202, an air pump 203, an airbag 204, and a pressure sensor 205. These components may be connected through a communications line 206. The electronic device 200 further includes a wristband 207. A user may wear the electronic device 200 on a wrist by using the wristband 207.

In this embodiment, the MCU 201 may be configured to: control and process information, and is responsible for detecting a signal and controlling another component.

The storage unit 202 may be configured to store a preset pressure value. The preset pressure value is a possible pressure value of the airbag existing when a pulse wave signal occurs, and may be determined based on a large amount of sample data of the user. The electronic device 200 may determine, based on the preset pressure value, whether the pulse wave signal occurs. In some embodiments, the storage unit 202 may be further configured to store a preset first slope and a preset second slope, so that the electronic device 200 can determine, based on the first slope and the second slope, a range in which wearing tightness can be quantized. In some other embodiments, the storage unit 202 may be further configured to store a preset first time period and a preset second time period. The first time period and the second event may be used to provide the electronic device 200 with a range in which wearing tightness can be quantized.

The air pump 203 may inflate and pressurize the airbag 204 or deflate the airbag 204 under control of the MCU 201.

The airbag 204 may be made of polyvinyl chloride or silica gel. The airbag 204 is flat without inflation, and once the airbag 204 is inflated, the airbag 204 slowly bulges and presses the radial artery of the wrist. In this case, there may be a correspondence between a change of a pressure value of the airbag 204 and a pulsation of the radial artery of the wrist. At least two connection holes are disposed on the airbag 204. One connection hole (for example, referred to as a connection hole 1) may be configured to connect to the air pump 203, to implement inflation or deflation of the airbag 204, and the other connection hole (for example, referred to as a connection hole 2) may be configured to connect to the pressure sensor 205, so that the pressure sensor 205 picks up the pressure value of the airbag 204 in real time.

It may be understood that, because the pressure sensor 205 is connected to the airbag 204 through the connection hole 2, the pressure value picked up by the pressure sensor 205 is strictly a pressure value of the airbag 204 at the connection hole 2. A pressure value in the vicinity of the connection hole 1 that is in the airbag 204 and that is configured to connect to the air pump 203 is larger, and a pressure value in an area far from the connection hole 1 is smaller. However, because a size of the airbag 204 is small, distribution of pressure values of the airbag 204 differs very slightly. Therefore, in this embodiment of this application, it may be considered that the pressure values of the airbag 204 are evenly distributed. To be specific, in this embodiment, it may be considered that the pressure value that is of the airbag 204 at the connection hole 2 and that is picked up by the pressure sensor 205 is the pressure value of the airbag 204.

The MCU 201 may also be referred to as a processor, and may include one or more processing units, for example, may include an application processor (application processor, AP), a graphics processing unit (graphics processing unit, GPU), an image signal processor (image signal processor, ISP), a controller, a memory, a digital signal processor (digital signal processor, DSP), a baseband processor, and/or a neural-network processing unit (neural-network processing unit, NPU). Different processing units may be independent devices, or may be integrated into one or more processors. For example, the MCU 201 may receive the pressure value that is of the airbag 204 and that is transmitted by the pressure sensor 205, and determine a pressure signal based on a relationship between the pressure value and time, for example, determine a first pressure signal or a second pressure signal.

The storage unit 202 may also be referred to as an internal memory, and may be configured to store executable program code of the electronic device, where the executable program code includes instructions. The MCU 201 performs various function applications of the electronic device and data processing by running the instructions stored in the storage unit 202. For example, in this embodiment of this application, the MCU 201 may execute the instructions stored in the storage unit 202, and after receiving a blood pressure measurement operation, perform a corresponding event in response to the operation. For example, The MCU 201 may quantify the wearing tightness based on a slope or a time period that is of a curve of an original signal in an initial pressurization phase and that is collected before the pressure value of the airbag 204 reaches the preset pressure value, and correct a blood pressure measurement value based on the wearing tightness. The storage unit 202 may include a program storage area and a data storage area. The program storage area may store an operating system, an application required by at least one function (for example, a sound playing function or an image playing function), and the like. The data storage region may store data (for example, a pressure signal) created during use of the electronic device, and the like. In addition, the storage unit 202 may include a high-speed random access memory, and may further include a nonvolatile memory, for example, at least one magnetic disk storage device, a flash memory device, or a universal flash storage (universal flash storage, UFS).

It may be understood that a connection relationship between the modules that is shown in this embodiment is merely an example for description, and does not constitute a limitation on the structure of the electronic device. In some other embodiments, the electronic device may alternatively use a connection manner different from that in the foregoing embodiment, or a combination of a plurality of connection manners.

It addition, the structure shown in this embodiment does not constitute a specific limitation on the electronic device. In other embodiments, the electronic device may include more or fewer components than those shown in the figure, or combine some components, or split some components, or have different component arrangements. The components shown in the figure may be implemented by hardware, software, or a combination of software and hardware.

For example, the electronic device may further include one or more of the following modules: a charging management module, a power management module, a mobile communications module, and a wireless communications module. The charging management module is configured to receive a charging input from a charger, where the charger may be a wireless charger or a wired charger. In some embodiments of wired charging, the charging management module may receive a charging input of a wired charger through a USB port. In some embodiments of wireless charging, the charging management module may receive a wireless charging input through a wireless charging coil of the electronic device. When charging a battery of the electronic device, the charging management module may further supply power to the electronic device by using the power management module.

The power management module is configured to connect to the battery of the electronic device. The power management module receives an input from the battery and/or the charging management module, and supplies power to the MCU 201, the storage unit 202, the air pump control unit, and the like. The power management module may be further configured to monitor parameters such as a battery capacity, a battery cycle count, and a battery health status (electric leakage or impedance). In some other embodiments, the power management module may alternatively be disposed in a processor. In some other embodiments, the power management module and the charging management module may alternatively be disposed in a same device.

The electronic device may further have a wireless communication function. The wireless communication function of the electronic device may be implemented through an antenna 1, an antenna 2, the mobile communications module, the wireless communications module, a modem processor, a baseband processor, and the like.

The antenna 1 and the antenna 2 are configured to: transmit and receive electromagnetic wave signals. Each antenna of the electronic device may be configured to cover one or more communication frequency bands. Different antennas may be further multiplexed, to improve antenna utilization. For example, the antenna 1 may be multiplexed as a diversity antenna in a wireless local area network. In some other embodiments, an antenna may be used in combination with a tuning switch.

The mobile communications module may provide a wireless communication solution that is applied to the electronic device and that includes the 3rd generation (3rd Generation, 3G) mobile communications technology/the 4th generation (4th Generation, 4G) mobile communications technology/the 5th generation (5th Generation, 5G) mobile communications technology. The mobile communications module may include at least one filter, a switch, a power amplifier, a low noise amplifier (low noise amplifier, LNA), and the like. The mobile communications module may receive an electromagnetic wave through the antenna 1, perform processing such as filtering or amplification on the received electromagnetic wave, and transmit the electromagnetic wave to the modem processor for demodulation. The mobile communications module may further amplify a signal modulated by the modem processor, and convert the signal into an electromagnetic wave for radiation through the antenna 1. In some embodiments, at least some functional modules in the mobile communications module may be disposed in the processor. In some embodiments, at least some functional modules in the mobile communications module may be disposed in a same device as at least some modules in the processor.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor, and then transmitted to the application processor. The application processor outputs a sound signal by using an audio device (which is not limited to a speaker, a receiver, or the like) of the electronic device, or displays an image or a video through a display of the electronic device. In some embodiments, the modem processor may be an independent component. In some other embodiments, the modem processor may be independent of the processor, and is disposed in a same device as the mobile communications module or another functional module.

The wireless communications module may provide a wireless communication solution that is applied to the electronic device and that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communications module may be one or more devices integrating at least one communications processor module. The wireless communications module receives an electromagnetic wave through the antenna 2, performs frequency modulation and filtering processing on an electromagnetic wave signal, and sends a processed signal to the processor. The wireless communications module may further receive a to-be-sent signal from the processor, perform frequency modulation and amplification on the signal, and convert a processed signal into an electromagnetic wave for radiation through the antenna 2.

In some embodiments, the antenna 1 and the mobile communications module in the electronic device are coupled, and the antenna 2 and the wireless communications module in the electronic device are coupled, so that the electronic device can communicate with a network and another device by using a wireless communications technology. The wireless communications technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-CDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation system, SBAS).

The electronic device may further have a display function. For example, the electronic device may implement the display function by using the GPU, the display, the application processor, and the like. The GPU is a microprocessor for image processing, and connects the display to the application processor. The GPU is configured to: perform mathematical and geometric calculation, and render an image. The processor may include one or more GPUs that execute program instructions to generate or change display information.

The display is configured to display an image, a video, or the like. The display includes a display panel. The display panel may be a liquid crystal display (liquid crystal display, LCD), an organic light-emitting diode (organic light-emitting diode, OLED), an active-matrix organic light-emitting diode (active-matrix organic light emitting diode, AMOLED), a flexible light-emitting diode (flexible light-emitting diode, FLED), a mini LED, a micro LED, a micro OLED, a quantum dot light-emitting diode (quantum dot light emitting diode, QLED), or the like. In some embodiments, the electronic device may include one or N displays, where N is a positive integer greater than 1.

The electronic device may further have a photographing function, for example, may implement the photographing function by using the ISP, a camera, a video codec, the GPU, the display, the application processor, and the like.

The ISP is configured to process data fed back by the camera. The camera is configured to capture a static image or a video. An optical image of an object is generated through a lens, and is projected onto a photosensitive element. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into a standard image signal in an RGB format, a YUV format, or the like. In some embodiments, the electronic device may include one or N cameras, where N is a positive integer greater than 1.

The digital signal processor is configured to process a digital signal, and may further process another digital signal in addition to the digital image signal. For example, when the electronic device selects a frequency, the digital signal processor is configured to perform Fourier transform on frequency energy.

The video codec is configured to: compress or decompress a digital video. The electronic device may support one or more video codecs. In this way, the electronic device can play or record videos in a plurality of encoding formats, for example, moving picture experts group (moving picture experts group, MPEG)-1, MPEG-2, MPEG-3, and MPEG-4.

The NPU is a neural-network (neural-network, NN) computing processor, quickly processes input information by referring to a structure of a biological neural network, for example, by referring to a transfer mode between human brain neurons, and may further continuously perform self-learning. The NPU may be used to implement an application such as intelligent cognition of the electronic device, for example, image recognition, facial recognition, speech recognition, and text understanding.

The electronic device may further include an external memory interface, which may be configured to connect to an external storage card, for example, a micro SD card, to extend a storage capability of the electronic device. The external storage card communicates with the processor through the external memory interface, to implement a data storage function. For example, files such as music and a video are stored in the external storage card.

The electronic device further implements audio functions, for example, may implement the audio functions, for example, music playing and recording, by using the audio module, the speaker, the receiver, a microphone, a headset jack, the application processor, and the like.

In addition to the pressure sensor 205, the electronic device may further include one or more of the following sensors: a gyro sensor, a magnetic sensor, an acceleration sensor, a distance sensor, an optical proximity sensor, an ambient light sensor, a fingerprint sensor, a temperature sensor, a touch sensor, and a bone conduction sensor. The gyro sensor may be configured to determine a motion posture of the electronic device. In some embodiments, an angular velocity of the electronic device around three axes (that is, x, y, and z axes) may be determined by using the gyro sensor. The gyro sensor may be used for image stabilization during photographing. The gyro sensor may also be used in navigation and somatic game scenarios. The pressure sensor is configured to measure barometric pressure. In some embodiments, the electronic device calculates an altitude based on a value of the barometric pressure measured by the pressure sensor, to assist in positioning and navigation. The magnetic sensor includes a Hall sensor. The electronic device may detect opening and closing of a flip cover by using the magnetic sensor. The acceleration sensor may detect accelerations in various directions (usually on three axes) of the electronic device, and may detect a magnitude and a direction of gravity when the electronic device is stationary. The acceleration sensor may be further configured to identify a posture of the electronic device, and applied to an application such as switching between landscape mode and portrait mode or a pedometer. The distance sensor is configured to measure a distance. The electronic device may measure a distance through infrared or laser. For example, the optical proximity sensor may include a light-emitting diode (LED) and an optical detector, for example, a photodiode. The light-emitting diode may be an infrared light-emitting diode. The electronic device emits infrared light by using the light-emitting diode. The electronic device detects infrared reflected light from a nearby object by using the photodiode. When detecting sufficient reflected light, the electronic device may determine that there is an object near the electronic device. When detecting insufficient reflected light, the electronic device may determine that there is no object near the electronic device. The ambient light sensor is configured to sense ambient light brightness. The electronic device may adaptively adjust brightness of the display based on the sensed ambient light brightness. The fingerprint sensor is configured to collect a fingerprint. The electronic device may use a feature of the collected fingerprint to implement fingerprint-based unlocking, application lock access, fingerprint-based photographing, fingerprint-based call answering, and the like. The temperature sensor is configured to detect a temperature. The touch sensor is also referred to as a "touch panel". The touch sensor may be disposed on the display. The touch sensor and the display form a touchscreen that is also referred to as a "touch panel". The touch sensor is configured to detect a touch operation performed on or near the touch sensor. The touch sensor may transfer the detected touch operation to the application processor, to determine a type of a touch event. The display may provide a visual output related to the touch operation. In some other embodiments, the touch sensor may alternatively be disposed on a surface of the electronic device at a location different from that of the display. The bone conduction sensor may obtain a vibration signal. In some embodiments, the bone conduction sensor may obtain a vibration signal of a vibration bone of a human vocal part. The bone conduction sensor may further contact a body pulse to receive a blood pressure beating signal. In some embodiments, the bone conduction sensor may also be disposed in a headset, to obtain a bone conduction headset. The audio module may obtain a voice signal through parsing based on the vibration signal that is of the vibration bone of the vocal part and that is obtained by the bone conduction sensor, to implement a voice function. The application processor may parse heart rate information based on the blood pressure beating signal obtained by the bone conduction sensor, to implement a heart rate detection function.

The electronic device may further include an indicator, for example, may be an indicator light, which may be configured to indicate a charging status and a power change, or may be configured to indicate a message, a missed call, a notification, and the like. A SIM card interface is configured to connect to a SIM card. The SIM card may be inserted into a SIM card interface or removed from a SIM card interface, to implement contact with or separation from the electronic device. The electronic device may support one or N SIM card interfaces, where N is a positive integer greater than 1.

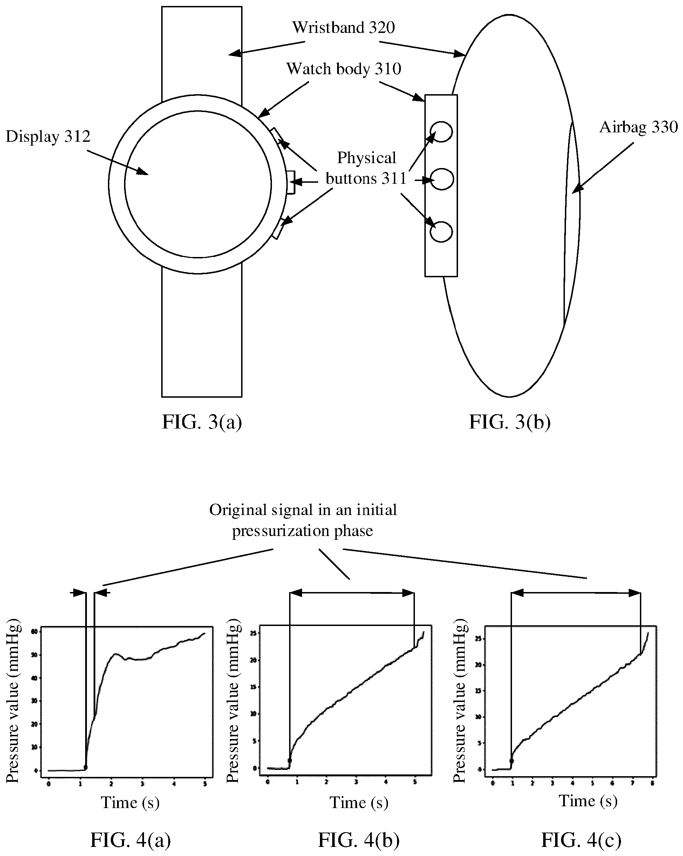

In this embodiment of this application, the electronic device shown in FIG. 2 may be implemented in a plurality of forms. For example, FIG. 3(a) and FIG. 3(b) are a schematic diagram of a product form of an electronic device according to an embodiment of this application. As shown in FIG. 3(a) and FIG. 3(b), for example, the electronic device is a blood pressure watch. FIG. 3(a) shows a front view of the blood pressure watch. FIG. 3(b) shows a side view of the blood pressure watch. The blood pressure watch may include a watch body 310, a wristband 320, and an airbag 330.

One or more physical buttons 311 may be disposed on the watch body 310. A user may trigger, by performing an operation on the physical button 311, the blood pressure watch to perform a corresponding event. For example, in some embodiments, the one or more physical buttons 311 include at least one physical button configured to trigger the blood pressure watch to start to measure blood pressure. When the user performs an operation on the physical button, for example, after the user presses the physical button, the blood pressure watch starts to measure blood pressure. Alternatively, the physical button 311 may not be disposed on the watch body 310.

A display 312 may be further disposed on the watch body 310. The display 312 may be configured to display related information to the user, for example, a blood pressure measurement result, or prompt the user to adjust wearing tightness of the blood pressure watch. The display 312 may be a touchscreen. The user can enter a related operation on the display 312. For example, in some embodiments, the user may enter a related operation on the display 312, for example, tap an icon displayed on the display 312 (where the icon may be an icon of an application that is installed in the blood pressure watch and that is used for blood pressure measurement, or another icon that can be used to trigger the blood pressure watch to measure blood pressure), to trigger the blood pressure watch to start to measure blood pressure.

The wristband 320 may be configured to strap the blood pressure watch to a wrist of the user. The airbag 330 is disposed inside the wristband 302. When the user wears the blood pressure watch on the wrist by using the wristband 320, the airbag 330 is attached to the radial artery of the user, and when the airbag 330 is inflated and a pressure value of the airbag 330 is close to a pressure value at the radial artery of the user, the blood pressure watch may measure the pressure value at the radial artery of the user based on a change of the pressure value of the airbag 330, to determine a blood pressure value of the user.

All methods in the following embodiments may be implemented in the electronic device having the foregoing hardware structure. Descriptions are provided below by using an example in which the electronic device is a wrist sphygmomanometer (for example, a blood pressure watch).

Usually, when measuring blood pressure, a difference in wearing tightness of the blood pressure watch significantly affects an original signal in an initial pressurization phase. FIG. 4(a), FIG. 4(b), and FIG. 4(c) are a schematic diagram of comparison between waveforms of an original signal in an initial pressurization phase with different wearing tightness. The original signal in the initial pressurization phase is a pressurization signal obtained through measurement when a pressure value of an airbag is less than or equal to a preset pressure value (for example, 20 mmHg). As shown in FIG. 4(a), when the blood pressure watch is worn tightly, because the airbag is already attached to the skin and is already under force, resistance to inflation of the airbag is very large, and consequently pressure of the airbag rises abruptly when a small amount of gas is filled. Therefore, the obtained original signal in the initial pressurization phase has a fast pressurization rate and short signal duration. As shown in FIG. 4(b), when wearing tightness of the blood pressure watch is appropriate, a pressure value of the airbag increases in a gentle curve as gas is filled, and signal duration is long. As shown in FIG. 4(c), when the blood pressure watch is worn loosely, the airbag needs to be first inflated to be attached to the skin, and pressure is exerted on the skin only after the airbag continues to be inflated. Resistance to inflation of the airbag needs to be far less than resistance existing after the airbag contacts with the skin. Therefore, the obtained original signal in the initial pressurization phase has a slow pressurization rate and long duration.

The initial pressurization phase may be a pressurization phase in which the pressure value of the airbag is less than the preset pressure value.

For example, the preset pressure value may be determined based on a possible pressure value of the airbag existing when a pulse wave signal occurs. In a process in which the blood pressure watch controls the air pump to start to inflate and pressurize the airbag until the pressure value of the airbag reaches the preset pressure value, because small pressure is exerted by the airbag on the radial artery, no significant compression effect is exerted on the radial artery. Therefore, a pulse wave signal cannot be extracted from the original signal within the time period. In this case, the original signal within the time period increases approximately linearly. Certainly, a time point at which the pulse wave signal occurs varies from person to person, and is not fixed. However, it may be determined by using a large amount of sample data of the user that, at a specific pressure value, no pulse wave signal occurs in original signals collected by most users when the users measure blood pressure. In this case, the pressure value may be set to the preset pressure value.

An embodiment of this application provides a blood pressure measurement method. A pressurization slope of a waveform curve is obtained from a waveform of an original signal in an initial pressurization phase, and the pressurization slope is used as a physical quantity for quantifying wearing tightness. In addition, a compensation value in this blood pressure measurement process is determined based on a mapping relationship between the pressurization slope of the waveform curve of the original signal in the initial pressurization phase and a compensation value of a blood pressure measurement value, and the blood pressure measurement value is corrected based on the compensation value, to obtain a more accurate blood pressure value.

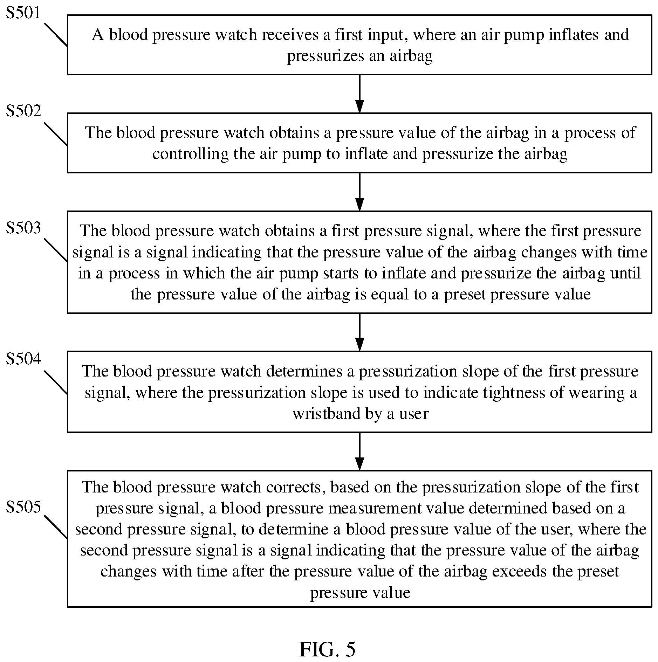

FIG. 5 is a schematic flowchart of a blood pressure measurement method according to an embodiment of this application. As shown in FIG. 5, the method may include S501 to S505.

S501: A blood pressure watch receives a first input, where an air pump inflates and pressurizes an airbag.

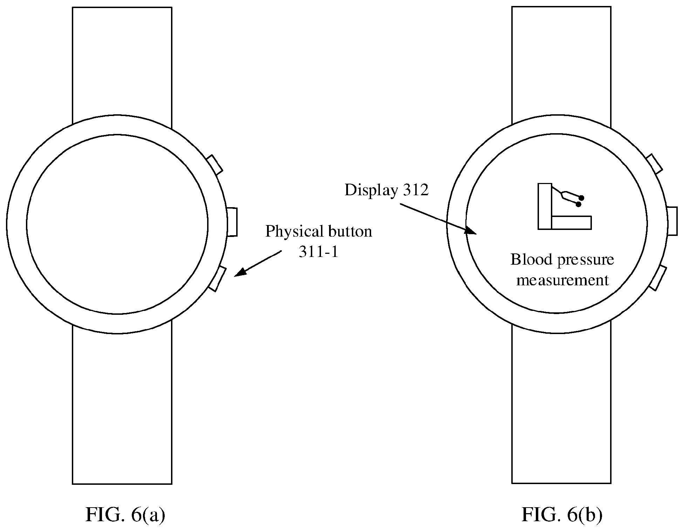

For example, when a user needs to perform blood pressure measurement by using the blood pressure watch worn on a wrist, the user may enter a corresponding operation (for example, the first input), to trigger the blood pressure watch to start to measure blood pressure. For example, refer to FIG. 6(a) and FIG. 6(b). When the user performs blood pressure measurement by using a blood pressure watch shown in FIG. 6(a), the user may press a physical button 311-1 disposed on the blood pressure watch, to trigger the blood pressure watch to start to measure blood pressure. For another example, when the user performs blood pressure measurement by using a blood pressure watch that includes a display 312 with a touch function and that is shown in FIG. 6(b), the user may touch a "Blood pressure measurement" icon on the display 312 of the blood pressure watch, to trigger the blood pressure watch to start to measure blood pressure. The "Blood pressure measurement" icon is an icon of an application that is installed in the blood pressure watch and that is used for blood pressure measurement. After receiving the operation, the blood pressure watch may control the air pump to inflate and pressurize the airbag, to start to measure blood pressure.

S502: The blood pressure watch obtains a pressure value of the airbag in a process of controlling the air pump to inflate and pressurize the airbag.

In the process of controlling the air pump to inflate and pressurize the airbag, the blood pressure watch may monitor a change of the pressure value of the airbag in real time.

For example, with reference to FIG. 2, in the process of controlling the air pump to inflate and pressurize the airbag, the pressure sensor 205 may obtain a pressure value of the airbag 204 in real time, and report the pressure value to the MCU 201, so that the MCU 201 monitors a change of the pressure value of the airbag 204 in real time. In some embodiments, the pressure value obtained by the pressure sensor 205 may be further stored in the storage unit 202, to subsequently determine blood pressure of the user.

S503: The blood pressure watch obtains a first pressure signal, where the first pressure signal is a signal indicating that the pressure value of the airbag changes with time in a process in which the air pump starts to inflate and pressurize the airbag until the pressure value of the airbag is equal to a preset pressure value.

The preset pressure value may be preset in the blood pressure watch, or may be configured by the user. The first pressure signal may be the original signal in the initial pressurization phase shown in FIG. 1.

For example, the blood pressure watch may detect, starting from inflating the airbag, whether the pressure value of the airbag reaches the preset pressure value. When the pressure value of the airbag reaches the preset pressure value, the blood pressure watch may use, as the first pressure signal, an original signal detected during a period from a time point at which the airbag starts to be inflated to a time point at which the pressure value of the airbag reaches the preset pressure value. For example, the preset pressure value may be in a range of 20 mmHg to 30 mmHg. In this embodiment of this application, the preset pressure value may be 20 mmHg. In this case, the first pressure signal may be an original signal detected by the blood pressure watch from a time point at which the blood pressure watch starts to inflate the airbag to a time point at which the pressure value of the airbag reaches 20 mmHg.

S504: The blood pressure watch determines a pressurization slope of the first pressure signal, where the pressurization slope is used to indicate tightness of wearing a wristband by the user.

The tightness of wearing the wristband by the user on the wrist is a subjective concept, and there is no unified physical quantity to measure the tightness. Based on FIG. 4, the pressurization slope of the curve of the first pressure signal may reflect the tightness of wearing the wristband by the user. Therefore, in this embodiment of this application, the pressurization slope may be used as a physical quantity for measuring the tightness of wearing the wristband by the user on the wrist. The pressurization slope may be determined by using a plurality of methods.

In some embodiments, an average slope of the curve of the first pressure signal may be used as the pressurization slope.

For example, the blood pressure watch may obtain the average slope through calculation by using Formula (1).

$$K_{ave} = A/B \qquad \text{Formula (1)}$$

Herein, $K_{ave}$ is the average slope of the curve of the first pressure signal, A is largest pressure in the curve of the first pressure signal, that is, the preset pressure value, and B is duration from a time point at which the airbag starts to be inflated and pressurized to a time point at which the pressure value of the airbag reaches the preset pressure value, that is, a pressurization time period. In this way, the blood pressure watch may use $K_{ave}$ determined based on the first pressure signal as the pressurization slope.

In some other embodiments, a largest instantaneous slope in the curve of the first pressure signal may be used as the pressurization slope.

For example, the blood pressure watch may randomly select a plurality of points in the curve of the first pressure signal, for example, $P_1$ to $P_n$, where n is an integer greater than 1, calculate an instantaneous slope at each of the n points, and use a largest instantaneous slope as the pressurization slope. The instantaneous slope may be obtained through calculation by using Formula (2).

$$K_n = A_n/B_n \qquad \text{Formula (2)}$$

Herein, $K_n$ is an instantaneous slope at an $n^{th}$ point, $A_n$ is a pressure value at the $n^{th}$ point, and $B_n$ is a time point corresponding to the $n^{th}$ point.

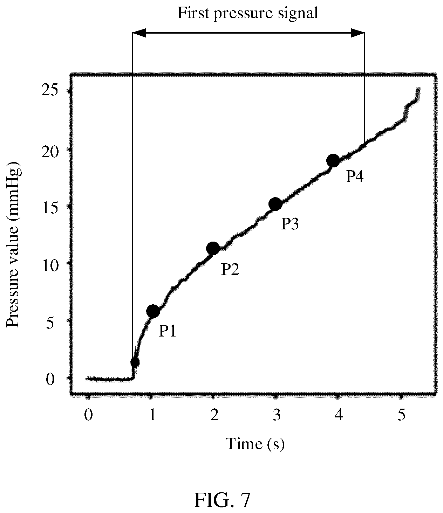

For example, as shown in FIG. 7, if n = 4, a total of four points: $P_1$ to $P_4$ are selected in the curve of the first pressure signal. The blood pressure watch determines that in the curve of the first pressure signal, a pressure value at the point $P_1$ is $A_1$, and a time point corresponding to the point $P_1$ is $B_1$. In this case, an instantaneous slope $K_1$ at the point $P_1$ may be $A_1/B_1$ according to Formula (1). Similarly, a pressure value at the point $P_2$ is $A_2$, and a time point corresponding to the point $P_2$ is $B_2$. In this case, an instantaneous slope at the point $P_2$ is $A_2/B_2$. A pressure value at the point $P_3$ is $A_3$, and a time point corresponding to the point $P_3$ is $B_3$. In this case, an instantaneous slope at the point $P_3$ is $A_3/B_3$. A pressure value at the point $P_4$ is

$A_4$, and a time point corresponding to the point $P_4$ is $B_4$. In this case, an instantaneous slope at the point $P_4$ is $A_4/B_4$. In this way, the blood pressure watch may select a largest value from $K_1, K_2, K_3,$ and $K_4$ as the pressurization slope.

In some other embodiments, the blood pressure watch may use, as the pressurization slope, an average value of several larger slopes in average slopes of curves between a plurality of adjacent points in the curve of the first pressure signal.

For example, the blood pressure watch may randomly select a plurality of points in the curve of the first pressure signal, for example, $P_1$ to $P_n$, where n is an integer greater than 1. The blood pressure watch separately calculates an average slope of a curve between two adjacent points in the n points, sorts a plurality of obtained slopes in descending order, and obtains an average value of the first m slopes, where m is a positive integer less than n. In this case, the average slope may be used as the pressurization slope. The average slope of the curve between the two adjacent points may be obtained through calculation by using Formula (3).

$$K_i = (B_{i+1} - B_i)/(A_{i+1} - A_i) \qquad \text{Formula (3)}$$

Herein, $K_i$ is an average slope of a curve between an $i^{th}$ point and an $(i+1)^{th}$ point in the n points, $B_i+1$ is a pressure value at the $(i+1)^{th}$ point, $B_i$ is a pressure value at the $i^{th}$ point, $A_i+1$ is a time point corresponding to the $(i+1)^{th}$ point, and $A_i$ is a time point corresponding to the $i^{th}$ point.

When the pressurization slope is determined based on instantaneous slopes at a plurality of points or slopes of curves between a plurality of points, a more accurate result is finally obtained when more points are selected. In an actual implementation process, a quantity of points may be flexibly selected based on a requirement. This is not limited in this embodiment of this application.

In this embodiment of this application, in the method in which the average slope of the curve of the first pressure signal is used as the pressurization slope, a calculation process in which a pressurization slope in a current blood pressure test process can be determined by performing only one time of calculation is simple, which does not cause excessive processing load on the blood pressure watch. In the method in which the largest instantaneous slope in the curve of the first pressure signal is used as the pressurization slope, when users with different wrist types and different wrist circumferences measure blood pressure, average slopes of curves of first pressure signals may differ slightly, but largest instantaneous slopes in the curves of the first pressure signals differ greatly. Therefore, the users with different wrist types and different wrist circumferences may use the largest instantaneous slopes as the pressurization slopes, so that the blood pressure watch can accurately quantify the wearing tightness. In the method in which the average value of the several larger slopes in the average slopes of the curves between the plurality of adjacent points in the curve of the first pressure signal is used as the pressurization slope, inaccurate quantization of the wearing tightness that is caused by an abnormal instantaneous slope (for example, incorrect data collection) at a location of an individual point in the curve of the first pressurization signal can be avoided. In actual application, flexible selection may be performed based on a requirement. This is not limited in this embodiment of this application.

In addition, it should be noted that, after a large quantity of experiments, the following conclusion can be obtained: There is a linear quantization space for quantifying the wearing tightness as the pressurization slope, that is, a method for quantifying the wearing tightness based on the pressurization slope is accurate only when the wearing tightness of wearing the wristband on the wrist of the user falls within a specific interval. Within this interval, there is a linear relationship between the pressurization slope and a blood pressure measurement offset caused by the wearing tightness. However, beyond this interval, this linear relationship is broken, the wearing tightness cannot be accurately quantified based on the pressurization slope, and a blood pressure offset is reversely deduced. Therefore, in this embodiment of this application, a first slope and a second slope may be set in the blood pressure watch, where the first slope is less than the second slope. [first slope, second slope] may be used to calibrate the foregoing interval. The first slope and the second slope may be preset in the blood pressure watch, or may be configured by the user. This is not limited in this embodiment of this application. In this embodiment of this application, the first slope and the second slope may be determined based on a large amount of sample data, and are preset in the blood pressure watch, or may be set by the user based on an actual situation.

Therefore, when the pressurization slope is not within a range of [first slope, second slope], some deviations may occur due to correction of the blood pressure measurement value based on the pressurization slope, resulting in an inaccurate result. Therefore, in some designs, different processing may be performed based on different cases of whether the pressurization slope falls within the range of [first slope, second slope] (for example, the pressurization slope is less than the first slope, the pressurization slope is greater than the first slope and less than the second slope, or the pressurization slope is greater than the second slope).

For example, in some embodiments, after the blood pressure watch determines the pressurization slope, if the pressurization slope is greater than the first slope and less than the second slope, it indicates that the wearing tightness may be quantized based on the pressurization slope, and the blood pressure measurement value may be corrected accordingly. In this case, the blood pressure watch may perform S505.

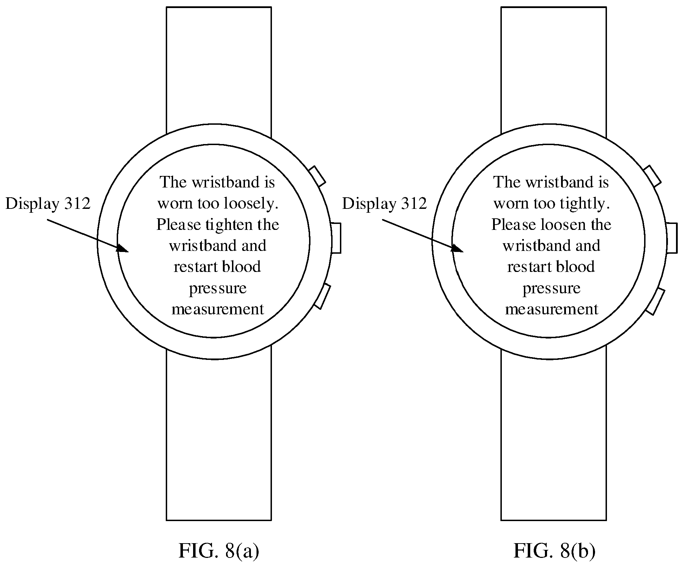

If the pressurization slope is less than the first slope, it indicates that the wristband is worn too loosely, and the wearing tightness cannot be accurately quantified based on the pressurization slope. In this case, the blood pressure watch may stop the measurement. For example, when the pressurization slope is less than the first slope, the blood pressure watch controls the air pump to stop inflating the airbag, and notifies the user that the blood pressure watch is worn too loosely during the measurement and prompts the user to tighten the wristband and restart the measurement. For example, as shown in FIG. 8(a), the blood pressure watch may display prompt information on the display 312, where the prompt information may be text information shown in FIG. 8(a), for example, "The wristband is worn too loosely. Please tighten the wristband and restart blood pressure measurement". The blood pressure watch may further display other information on the display 312, for example, a dynamic image of tightening the wristband, to remind the user to tighten the wristband and restart blood pressure measurement. For another example, the blood pressure watch includes a motor. The blood pressure watch may control the motor to vibrate at a preset frequency, for example, vibrate at a low frequency, to notify the user that the wristband is currently worn too loosely, and prompt the user to tighten the wristband and restart blood pressure measurement. For another example, the blood pressure watch includes an indicator light. The blood pressure watch may control the indicator light to blink at a preset frequency, for example, blink at a low frequency, to notify the user that the wristband is currently worn too loosely, and prompt the user to tighten the wristband and restart blood pressure measurement.

If the pressurization slope is greater than the second slope, it indicates that the wristband is worn too tightly, and the wearing tightness cannot be accurately quantified based on the pressurization slope. In this case, the blood pressure watch may stop the measurement. For example, when the pressurization slope is greater than the second slope, the blood pressure watch controls the air pump to stop inflating the airbag, and notifies the user that the blood pressure watch is worn too tightly during the measurement and prompts the user to loosen the wristband and restart the measurement. In this way, inaccurate blood pressure measurement caused because the blood pressure watch is worn too loosely or too tightly can be avoided, and the user is prompted to perform corresponding adjustment on the wearing tightness of the blood pressure watch, so that blood pressure measurement can be accurately performed. For example, as shown in FIG. 8(b), the blood pressure watch may display prompt information on the display 312, where the prompt information may be text information shown in FIG. 8(b), for example, "The wristband is worn too tightly. Please loosen the wristband and restart blood pressure measurement". The blood pressure watch may further display other information on the display 312, for example, a dynamic image of loosening the wristband, to remind the user to loosen the wristband and restart blood pressure measurement. For another example, the blood pressure watch includes a motor. The blood pressure watch may control the motor to vibrate at a preset frequency, for example, vibrate at a high frequency, to notify the user that the wristband is currently worn too tightly, and prompt the user to loosen the wristband and restart blood pressure measurement. For another example, the blood pressure watch includes an indicator light. The blood pressure watch may control the indicator light to blink at a preset frequency, for example, blink at a high frequency, to notify the user that the wristband is currently worn too tightly, and prompt the user to loosen the wristband and restart blood pressure measurement.

S505: The blood pressure watch corrects, based on the pressurization slope of the first pressure signal, a blood pressure measurement value determined based on a second pressure signal, to determine a blood pressure value of the user, where the second pressure signal is a signal indicating that the pressure value of the airbag changes with time after the pressure value of the airbag exceeds the preset pressure value.

As shown in FIG. 1, after the pressure value of the airbag exceeds the preset pressure value, the pressure value of the airbag is already close to the blood pressure value of the user. In this case, the blood pressure watch may extract a pulse wave signal from the original signal in a short time period. To be specific, the blood pressure watch may extract the pulse wave signal from the second pressure signal obtained after the pressure value of the airbag exceeds the preset pressure value.

The pulse wave signal may be obtained by performing filtering on the original signal. For example, filtering may be performed on the original signal by using a Butterworth filter (Butterworth filter) or a finite impulse response (Finite Impulse Response, FIR) filter to obtain the pulse wave signal. Usually, an envelope of the extracted pulse wave signal presents a single-peak characteristic of first increasing and then decreasing, but a pulse wave envelope of each of a small quantity of users also presents a double-peak or another waveform. After obtaining the pulse wave signal, the blood pressure watch may extract related characteristic information from the pulse wave signal. For example, the characteristic information may include envelope peak pressure, maximum slope static pressure, and the like. Further, the blood pressure watch may determine the blood pressure measurement value of the user based on the characteristic information.

Based on the foregoing descriptions, different offsets of the blood pressure measurement value are generated due to different tightness of wearing the wristband on the wrist of the user. According to the blood pressure measurement method provided in this embodiment of this application, the blood pressure measurement value may be corrected based on the quantized wearing tightness (for example, the pressurization slope), to obtain a more accurate blood pressure value.

For example, when the wearing tightness is quantized based on the pressurization slope, a compensation value in this measurement process may be determined based on a mapping relationship between the pressurization slope and the compensation value. To be specific, the blood pressure watch may use, as the compensation value in the measurement, a compensation value corresponding to the pressurization slope determined based on the first pressure signal, and correct the blood pressure measurement value based on the compensation value.

In this embodiment of this application, a mapping relationship between an SBP compensation value or a DBP compensation value and a pressurization slope may be obtained by collecting statistics about a large quantity of samples. For example, the mapping relationship in the blood pressure watch may be implemented as follows: The mapping relationship includes a table of a correspondence between the pressurization slope and the SBP compensation value or between the pressurization slope and the DBP compensation value, or may be a curve that can reflect a correspondence between the pressurization slope and the SBP compensation value or between the pressurization slope and the DBP compensation value. In the blood pressure measurement process, the blood pressure watch may determine, based on the mapping relationship between the SBP compensation value and the pressurization slope, an SBP compensation value corresponding to the pressurization slope in the blood pressure measurement, and correct an SBP measurement value based on the SBP compensation value. Descriptions are provided below by using an example in which a mapping relationship is a curve including a correspondence between a pressurization slope and an SBP compensation value.

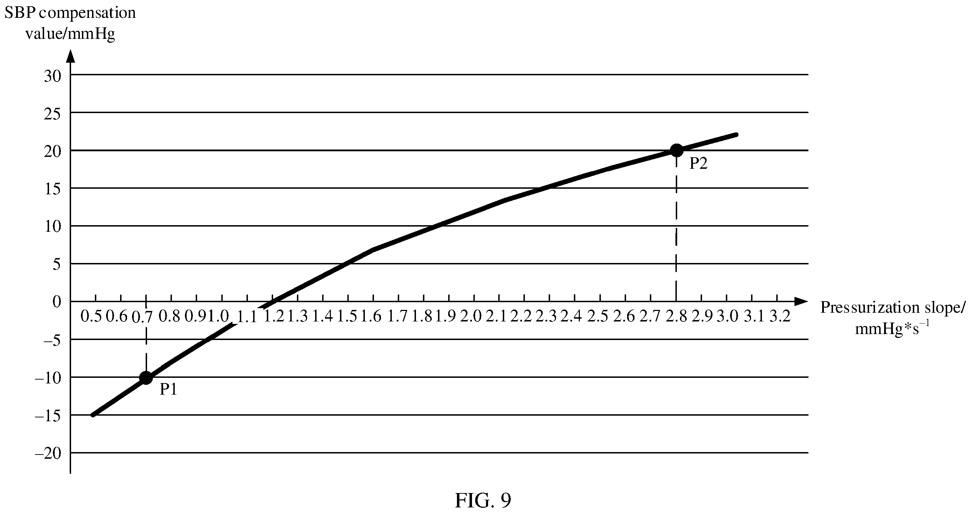

As shown in FIG. 9, when the wearing tightness falls within a specific range (that is, the wristband is not worn too loosely or too tightly), the SBP compensation value is approximately positively correlated with the pressurization slope. When the blood pressure watch is worn loosely, the pressurization slope is small, and a corresponding compensation value is negative. As the blood pressure watch is worn more tightly, the pressurization slope gradually increases. When the blood pressure watch is in a tight state, a corresponding compensation value is positive.

For example, when the blood pressure watch determines that the pressurization slope is 0.7 mmHg/s, corresponding to a point P1 in the curve shown in FIG. 9, the blood pressure watch may determine that the SBP compensation value is -10. This indicates that an actual SBP value is 10 mmHg less than a measured SBP value because the blood pressure watch is worn too loosely. Therefore, corrected SBP is a value obtained by subtracting 10 mmHg from the measured SBP. For another example, when the blood pressure watch determines that the pressurization slope is 2.8 mmHg/s, corresponding to a point P2 in the curve shown in FIG. 9, the blood pressure watch may determine that the SBP compensation value is 20. This indicates that an actual SBP value is 20 mmHg greater than a measured SBP value because the blood pressure watch is worn too tightly. Therefore, corrected SBP is a value obtained by adding 20 mmHg to the measured SBP.

Similarly, the blood pressure watch may determine, based on the mapping relationship between the DBP compensation value and the pressurization slope, a DBP compensation value corresponding to the pressurization slope in the blood pressure measurement, and correct a DBP measurement value based on the DBP compensation value.

It should be noted that mapping relationships between compensation values and pressurization slopes are correspondingly different due to different materials, widths, and structures of airbags. In a design phase of the blood pressure watch, after a hardware specification of the blood pressure watch is determined, a mapping relationship that is between a pressurization slope and each of a DBP compensation value and an SBP compensation value and that corresponds to the hardware specification may be obtained by collecting statistics about and analyzing a large quantity of samples.

In this way, the electronic device quantizes the wearing tightness of the user based on the pressurization slope of the first pressure signal, and determines a compensation value of a measurement result based on the quantization result, to compensate for the measurement result, so as to obtain a more accurate blood pressure measurement value that is not affected by a difference in the wearing tightness. In addition, the electronic device further determines, based on whether the quantized wearing tightness (for example, the pressurization slope) falls within a preset interval, whether there is a problem that the blood pressure watch is worn too loosely or too tightly. If there is the foregoing problem, the user may be prompted to adjust the wearing tightness, to further improve blood pressure measurement accuracy. Because the wearing tightness is strongly related to a use habit and feelings of each user, a fixed wearing method cannot accurately adapt to all users. According to the blood pressure measurement method provided in this embodiment of this application, the wearing tightness can be quantified based on a change of the pressure value of the airbag. In the method, impact of current wearing tightness on blood pressure measurement is accurately determined based on the change of the pressure value of the airbag in a case of a current wrist type and wrist circumference of the user, so that the method can adapt to users with different wrist types and wrist circumferences.

An embodiment of this application further provides a blood pressure measurement method. An electronic device may determine a pressurization time period from a time point at which an airbag starts to be pressurized to a time point at which a pressure value of the airbag reaches a preset pressure value, and use the pressurization time period as a physical quantity for quantifying wearing tightness. Further, the blood pressure watch may determine a compensation value in this blood pressure measurement process based on a mapping relationship between the pressurization time period and a compensation value of a blood pressure measurement value, and may compensate for the blood pressure measurement value based on the compensation value, to obtain a more accurate blood pressure value of a user.

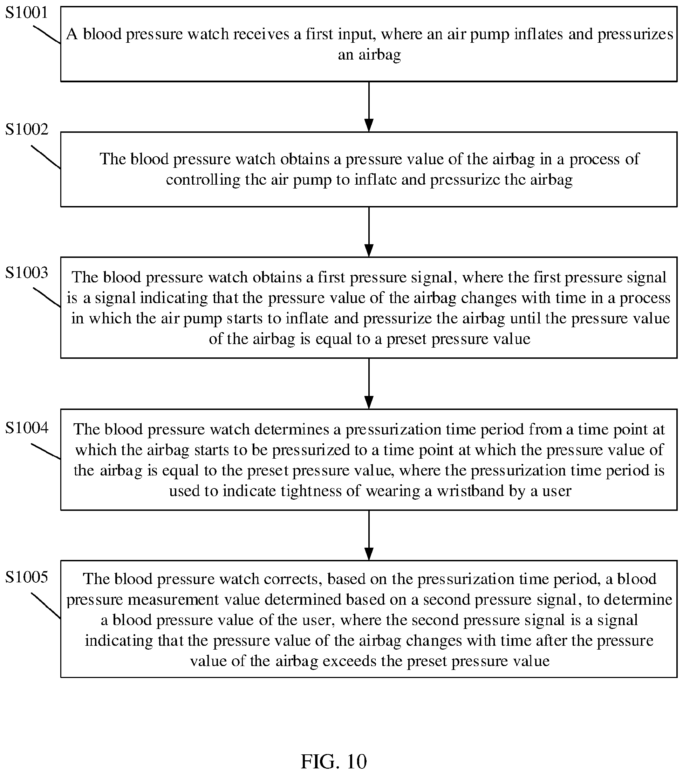

FIG. 10 is a schematic flowchart of another blood pressure measurement method according to an embodiment of this application. As shown in FIG. 10, the method may include S1001 to S1005.

S1001 to S1003 are respectively the same as S501 to S503 in FIG. 5. Details are not described herein in this embodiment. The following describes in detail S1004 and S1005 that are different from those in FIG. 5.

S 1004: The blood pressure watch determines a pressurization time period from a time point at which the airbag starts to be pressurized to a time point at which the pressure value of the airbag is equal to the preset pressure value, where the pressurization time period is used to indicate tightness of wearing a wristband by the user.

The tightness of wearing the wristband by the user on the wrist is a subjective concept, and there is no unified physical quantity to measure the tightness. When the user wears the wristband more tightly, a time period (that is, the pressurization time period) from a time point at which the pressure value of the airbag starts to be increased to a time point at which the pressure value of the airbag reaches the preset pressure value is shorter. On the contrary, when the user wears the wristband more loosely, the pressurization time period is longer. Therefore, in this embodiment of this application, the pressurization time period may be used as a physical quantity for measuring the tightness of wearing the wristband by the user on the wrist.

In some embodiments, the blood pressure watch may determine, based on the first pressure signal (that is, the original signal in the initial pressurization phase), duration from a time point at which the first pressure signal occurs to a time point at which the first pressure signal ends, where the duration is the pressurization time period.

In some other embodiments, the blood pressure watch may alternatively use, as the pressurization time period, a difference between a time point at which the pressure value of the airbag reaches the preset pressure value and a time point at which the pressure value of the airbag starts to be increased.

In addition, after a large quantity of experiments, a conclusion similar to that in S503 may be obtained: A method for quantifying the wearing tightness based on the pressurization time period is accurate only when the wearing tightness of wearing the wristband on the wrist of the user falls within a specific interval. Within this interval, there is a linear relationship between the pressurization time period and a blood pressure measurement offset caused by the wearing tightness. However, beyond this interval, this linear relationship is broken, the wearing tightness cannot be accurately quantified based on the pressurization time period, and a blood pressure offset is reversely deduced. Therefore, in this embodiment of this application, a first time period and a second time period may be set in the blood pressure watch, to calibrate the foregoing interval. The first time period and the second time period may be preset in the blood pressure watch, or may be configured by the user. This is not limited in this embodiment of this application.

Therefore, when the pressurization time period is not within a range of [first time period, second time period], some deviations may occur due to correction of the blood pressure measurement value based on the pressurization time period, resulting in an inaccurate result. Therefore, in some designs, different processing may be performed based on different cases of whether the pressurization time period falls within the range of [first time period, second time period] (for example, the pressurization time period is less than the first time period, the pressurization time period is greater than the first time period and less than the second time period, or the pressurization slope is greater than the second time period).

In some embodiments, after determining the pressurization time period, the blood pressure watch may determine that the pressurization time period is greater than the first time period and less than the second time period. In this case, it indicates that the wearing tightness may be quantized based on the pressurization time period. In this case, the blood pressure watch may perform S1005.

If the pressurization time period is less than the first time period, it indicates that the wristband is worn too tightly, and the wearing tightness cannot be accurately quantified based on the pressurization time period. In this case, the blood pressure watch may stop the measurement. For example, when the pressurization time period is less than the first time period, the blood pressure watch controls the air pump to stop inflating the airbag, and notifies the user that the blood pressure watch is worn too tightly during the measurement and prompts the user to loosen the wristband and restart the measurement.

If the pressurization time period is greater than the second time period, it indicates that the wristband is worn too loosely, and the wearing tightness cannot be accurately quantified based on the pressurization time period. In this case, the blood pressure watch may stop the measurement. For example, when the pressurization time period is greater than the second time period, the blood pressure watch controls the air pump to stop inflating the airbag, and notifies the user that the blood pressure watch is worn too loosely during the measurement and prompts the user to tighten the wristband and restart the measurement. In this way, inaccurate blood pressure measurement caused because the blood pressure watch is worn too loosely or too tightly can be avoided, and the user is prompted to perform corresponding adjustment on the wearing tightness of the blood pressure watch, so that blood pressure measurement can be accurately performed.

S1005: The blood pressure watch corrects, based on the pressurization time period, a blood pressure measurement value determined based on a second pressure signal, to determine a blood pressure value of the user, where the second pressure signal is a signal indicating that the pressure value of the airbag changes with time after the pressure value of the airbag exceeds the preset pressure value.

As described in S505, different offsets of the blood pressure measurement value are generated due to different tightness of wearing the wristband on the wrist of the user. According to the blood pressure measurement method provided in this embodiment of this application, the blood pressure measurement value may be corrected based on the quantized wearing tightness (for example, the pressurization time period), to obtain a more accurate blood pressure value.

For example, when the wearing tightness is quantized based on the pressurization time period, a compensation value in this measurement process may be determined based on a mapping relationship between the pressurization time period and the compensation value. To be specific, the blood pressure watch may use, as the compensation value in the measurement, a compensation value corresponding to the pressurization time period, and correct the blood pressure measurement value based on the compensation value.

Similar to descriptions in S505, a mapping relationship between an SBP compensation value or a DBP compensation value and a pressurization time period may be obtained by collecting statistics about a large quantity of samples. For example, the mapping relationship in the blood pressure watch may be implemented as follows: The mapping relationship includes a table of a correspondence between the pressurization time period and the SBP compensation value or between the pressurization time period and the DBP compensation value, or may be a curve that can reflect a correspondence between the pressurization time period and the SBP compensation value or between the pressurization time period and the DBP compensation value. In the blood pressure measurement process, the blood pressure watch may determine, based on the mapping relationship between the SBP compensation value and the pressurization time period, an SBP compensation value corresponding to the pressurization time period in the blood pressure measurement, and correct an SBP measurement value based on the SBP compensation value. Descriptions are provided below by using an example in which a mapping relationship is a curve including a correspondence between a pressurization time period and an SBP compensation value.

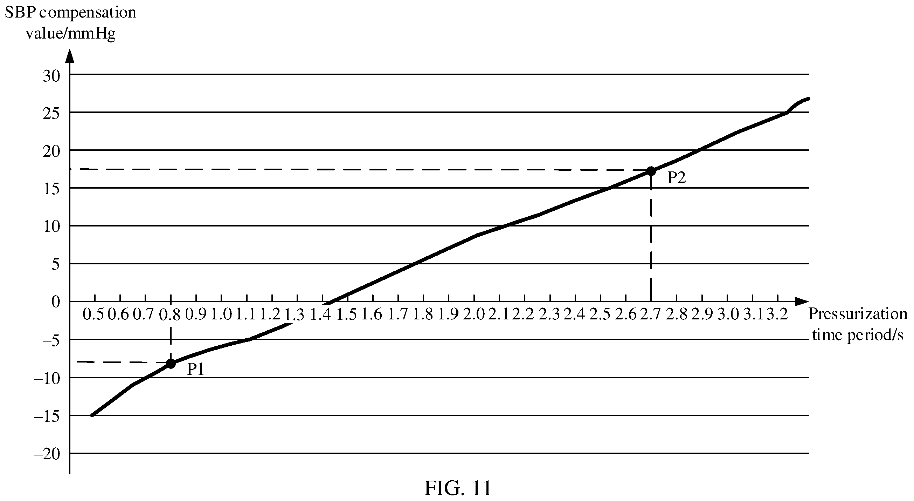

For example, as shown in FIG. 11, when the wearing tightness falls within a specific range (that is, the wristband is not worn too loosely or too tightly), the SBP compensation value is approximately positively correlated with the pressurization time period. When the blood pressure watch is worn tightly, the pressurization time period is short, and a corresponding compensation value is negative. As the blood pressure watch is worn more loosely, the pressurization time period gradually increases. When the blood pressure watch is in a loose state, a corresponding compensation value is positive.

For example, when the blood pressure watch determines that the pressurization time period is 0.8s, corresponding to a point P1 in the curve shown in FIG. 11, the blood pressure watch may determine that the SBP compensation value is -8 mmHg. This indicates that an actual SBP value is 8 mmHg greater than a measured SBP value because the blood pressure watch is worn too tightly. Therefore, corrected SBP is a value obtained by adding 8 mmHg to the measured SBP. For another example, when the blood pressure watch determines that the pressurization time period is 2.7s, corresponding to a point P2 in the curve shown in FIG. 11, the blood pressure watch may determine that the SBP compensation value is 17 mmHg. This indicates that an actual SBP value is 17 mmHg less than a measured SBP value because the blood pressure watch is worn too loosely. Therefore, corrected SBP is a value obtained by subtracting 17 mmHg from the measured SBP.

Similarly, the blood pressure watch may determine, based on the mapping relationship between the DBP compensation value and the pressurization time period, a DBP compensation value corresponding to the pressurization time period in the blood pressure measurement, and correct a DBP measurement value based on the DBP compensation value.

It should be noted that mapping relationships between compensation values and pressurization time period are correspondingly different due to different materials, widths, and structures of airbags. In a design phase of the blood pressure watch, after a hardware specification of the blood pressure watch is determined, a mapping relationship that is between a pressurization time period and each of a DBP compensation value and an SBP compensation value and that corresponds to the hardware specification may be obtained by collecting statistics about and analyzing a large quantity of samples.

In this way, the electronic device quantizes the wearing tightness of the user based on the pressurization time period of the first pressure signal, and determines a compensation value of a measurement result based on the quantization result, to compensate for the measurement result, so as to obtain a more accurate blood pressure measurement value that is not affected by a difference in the wearing tightness. In addition, the electronic device further determines, based on whether the quantized wearing tightness (for example, the pressurization time period) falls within a preset interval, whether there is a problem that the blood pressure watch is worn too loosely or too tightly. If there is the foregoing problem, the user may be prompted to adjust the wearing tightness, to further improve blood pressure measurement accuracy.

The foregoing mainly describes, from a perspective of the electronic device, the solutions provided in the embodiments of this application. It may be understood that, to implement the foregoing functions, the electronic device includes corresponding hardware structures and/or software modules for performing each function. The corresponding hardware structures and/or software modules for performing each function may constitute an electronic device. A person skilled in the art should be easily aware that algorithms and steps in the examples described with reference to the embodiments disclosed in this specification can be implemented in a form of hardware or a combination of hardware and computer software in this application. Whether a function is performed by hardware or hardware driven by computer software depends on particular applications and design constraints of the technical solutions. A person skilled in the art may use different methods to implement the described functions for each particular application, but it should not be considered that the implementation goes beyond the scope of this application.

In the embodiments of this application, the electronic device may be divided into functional modules based on the method examples. For example, each functional module may be obtained through division based on each corresponding function, or two or more functions may be integrated into one processing module. The integrated module may be implemented in a form of hardware, or may be implemented in a form of a software functional module. It should be noted that, in the embodiments of this application, division into the modules is an example and is merely logical function division, and may be other division in an actual implementation.

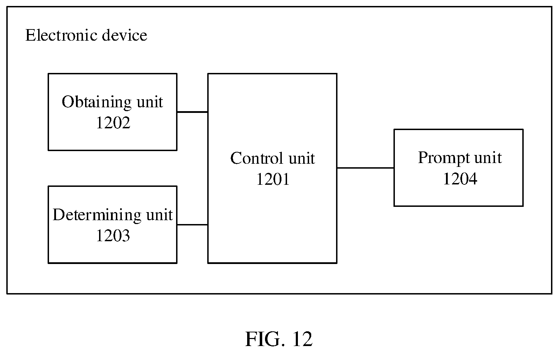

When each functional module is obtained through division based on each corresponding function, FIG. 12 is a possible schematic composition diagram of the electronic device in the foregoing embodiment. As shown in FIG. 12, the electronic device may include a control unit 1201, an obtaining unit 1202, and a determining unit 1203.

The control unit 1201 is configured to: when the electronic device is worn on a wrist of a user, control an air pump of the electronic device to inflate and pressurize an airbag of the electronic device. For example, the control unit 1201 may be configured to perform S501 in the blood pressure measurement method shown in FIG. 5. The control unit 1201 may be further configured to perform S1001 in the blood pressure measurement method shown in FIG. 10.

The obtaining unit 1202 is configured to obtain a pressure value of the airbag in a process of controlling the air pump to inflate and pressurize the airbag. For example, the obtaining unit 1202 may be configured to perform S502 in the blood pressure measurement method shown in FIG. 5. The obtaining unit 1202 may be further configured to perform S1002 in the blood pressure measurement method shown in FIG. 10.

The obtaining unit 1202 is further configured to obtain a first pressure signal, where the first pressure signal is a signal indicating that the pressure value of the airbag changes with time in a process in which the air pump starts to inflate and pressurize the airbag until the pressure value of the airbag is equal to a preset pressure value. For example, the obtaining unit 1202 may be configured to perform S503 in the blood pressure measurement method shown in FIG. 5. The obtaining unit 1202 may be further configured to perform S1003 in the blood pressure measurement method shown in FIG. 10.

The determining unit 1203 is configured to determine, based on the first pressure signal, tightness of wearing a wristband by the user. For example, the determining unit 1203 may be configured to perform S504 in the blood pressure measurement method shown in FIG. 5. The determining unit 1203 may be further configured to perform S1004 in the blood pressure measurement method shown in FIG. 10.

The determining unit 1203 is further configured to correct, based on the tightness, a blood pressure measurement value determined based on a second pressure signal, to determine a blood pressure value of the user, where the second pressure signal is a signal indicating that the pressure value of the airbag changes with time after the pressure value of the airbag exceeds the preset pressure value. For example, the determining unit 1203 may be configured to perform S505 in the blood pressure measurement method shown in FIG. 5. The determining unit 1203 may be further configured to perform S1005 in the blood pressure measurement method shown in FIG. 10.

In a possible design, the determining unit 1203 is configured to determine a pressurization slope of the first pressure signal, where the pressurization slope is a slope of a curve of the first pressure signal, and the pressurization slope may be used to indicate the tightness of wearing the wristband by the user. The determining unit 1203 is further configured to correct the blood pressure measurement value based on the pressurization slope of the first pressure signal, to determine the blood pressure value. For example, the determining unit 1203 may be configured to perform S504 and S505 in the blood pressure measurement method shown in FIG. 5.

In a possible design, the determining unit 1203 is further configured to determine a compensation value based on the pressurization slope of the first pressure signal and a mapping relationship, where the mapping relationship includes a mapping relationship between the pressurization slope of the first pressure signal and the compensation value. The determining unit 1203 is further configured to correct the blood pressure measurement value by using the compensation value, to determine the blood pressure value.

In a possible design, when determining that the pressurization slope is greater than a first slope and less than a second slope, the determining unit 1203 corrects the blood pressure measurement value based on the pressurization slope of the first pressure signal, to determine the blood pressure value.

In a possible design, the apparatus further includes a prompt unit 1204. The control unit 1201 is further configured to: when it is determined that the pressurization slope is less than the first slope, control the air pump to stop inflating and pressurizing the airbag. The prompt unit 1204 is configured to prompt the user to tighten the wristband. The control unit 1201 is further configured to: when it is determined that the pressurization slope is greater than the second slope, control the air pump to stop inflating and pressurizing the airbag. The prompt unit 1204 is configured to prompt the user to loosen the wristband.

In a possible design, the determining unit 1203 is configured to determine, based on the first pressure signal, a pressurization time period from a time point at which the airbag starts to be pressurized to a time point at which the pressure value of the airbag is equal to the preset pressure value, where the pressurization time period is used to indicate the tightness of wearing the wristband by the user. The determining unit 1203 is further configured to correct the blood pressure measurement value based on the pressurization time period, to determine the blood pressure value. For example, the determining unit 1203 may be configured to perform S 1004 and S1005 in the blood pressure measurement method shown in FIG. 10.

In a possible design, the determining unit 1203 is further configured to determine a compensation value based on the pressurization time period and a mapping relationship, where the mapping relationship includes a mapping relationship between the pressurization time period and the compensation value. The determining unit 1203 is further configured to correct the blood pressure measurement value by using the compensation value, to determine the blood pressure value.

In a possible design, when determining that the pressurization time period is greater than a first time period and less than a second time period, the determining unit 1203 corrects the blood pressure measurement value based on the pressurization time period, to determine the blood pressure value.

In a possible design, the apparatus further includes a prompt unit 1204. The control unit 1201 is further configured to: when it is determined that the pressurization time period is less than the first time period, control the air pump to stop inflating and pressurizing the airbag. The prompt unit 1204 is configured to prompt the user to tighten the wristband. The control unit 1201 is further configured to: when it is determined that the pressurization time period is greater than the second time period, control the air pump to stop inflating and pressurizing the airbag. The prompt unit 1204 is configured to prompt the user to loosen the wristband.

It should be noted that all related content in the steps in the foregoing method embodiments may be cited in function descriptions of corresponding functional modules. Details are not described herein again. The electronic device provided in this embodiment of this application is configured to perform the foregoing blood pressure measurement method. Therefore, a same effect as the foregoing blood pressure measurement method can be achieved.

Based on the foregoing descriptions of the implementations, a person skilled in the art may clearly understand that for the purpose of convenient and brief descriptions, division into the foregoing functional modules is merely used as an example for illustration. In actual application, the foregoing functions can be allocated to different functional modules for implementation based on a requirement, that is, an inner structure of an apparatus is divided into different functional modules to implement all or some of the functions described above.

In the several embodiments provided in this application, it should be understood that the disclosed apparatus and method may be implemented in another manner. For example, the described apparatus embodiment is merely an example. For example, division into the modules or units is merely logical function division and may be other division in an actual implementation. For example, a plurality of units or components may be combined or integrated into another apparatus, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or another form.

The units described as separate parts may or may not be physically separate, and parts displayed as units may be one or more physical units, in other words, may be located in one place, or may be distributed in different places. Some or all of the units may be selected based on actual requirements to achieve the objectives of the solutions in the embodiments.

In addition, functional units in the embodiments of this application may be integrated into one processing unit, each of the units may exist alone physically, or two or more units may be integrated into one unit. The integrated unit may be implemented in a form of hardware, or may be implemented in a form of a software functional unit.

When the integrated unit is implemented in a form of a software functional unit and sold or used as an independent product, the integrated unit may be stored in a readable storage medium. Based on such an understanding, the technical solutions of the embodiments of this application essentially, or the part contributing to the conventional technology, or all or some of the technical solutions may be implemented in a form of a software product. The software product is stored in a storage medium and includes several instructions for instructing a device (which may be a single-chip microcomputer, a chip, or the like) or a processor (processor) to perform all or some of the steps of the methods in the embodiments of this application. The foregoing storage medium includes any medium that can store program code, such as a USB flash drive, a removable hard disk, a read-only memory (Read-Only Memory, ROM), a random access memory (Random Access Memory, RAM), a magnetic disk, or an optical disc.

The foregoing descriptions are merely specific implementations of this application, but are not intended to limit the protection scope of this application. Any variation or replacement within the technical scope disclosed in this application shall fall within the protection scope of this application. Therefore, the protection scope of this application shall be subject to the protection scope of the claims.

**Claims**

1. A blood pressure measurement method, performed by
an electronic device, wherein the electronic device comprises a wristband, an airbag, and an air pump, and the electronic device is worn on a wrist of a user; and the method comprises:

receiving (S1001), by the electronic device, a first input;
controlling the air pump to inflate and pressurize the airbag, after receiving the first input;
obtaining (S1003), by the electronic device, a first pressure signal, wherein the first pressure signal is a signal indicating that a pressure value of the airbag changes with time in a process in which the air pump starts to inflate and pressurize the airbag to a preset pressure value;
determining, by the electronic device based on the first pressure signal, tightness of wearing the wristband by the user; and
correcting, by the electronic device based on the tightness, a blood pressure measurement value determined based on a second pressure signal, to determine a blood pressure value of the user, wherein the second pressure signal is a signal indicating that the pressure value of the airbag changes with time after the pressure value of the airbag exceeds the preset pressure value, wherein the determining, by the electronic device based on the first pressure signal, tightness of wearing the wristband by the user comprises:

determining (S1004), by the electronic device based on the first pressure signal, a pressurization time period from a time point at which the airbag starts to be pressurized to a time point at which the pressure value of the airbag is equal to the preset pressure value, wherein the pressurization time period is used to indicate the tightness of wearing the wristband by the user; and
the correcting, by the electronic device, a blood pressure measurement value based on the tightness, to determine a blood pressure value of the user comprises:
correcting (S1005), by the electronic device, the blood pressure measurement value based on the pressurization time period, to determine the blood pressure value of the user.

2. The method according to claim 1, wherein the correcting (S1005), by the electronic device, the blood pressure measurement value based on the pressurization time period, to determine the blood pressure value of the user comprises:

determining, by the electronic device, a compensation value based on a mapping relationship, wherein the mapping relationship comprises a mapping relationship between the pressurization time period and the compensation value; and
correcting, by the electronic device, the blood pressure measurement value by using the compensation value, to determine the blood pressure value of the user.

3. The method according to claim 1 or 2, wherein the correcting (S1005), by the electronic device, the blood pressure measurement value based on the pressurization time period, to determine the blood pressure value of the user comprises:
when the pressurization time period is greater than a first time period and less than a second time period, correcting, by the electronic device, the blood pressure measurement value based on the pressurization time period, to determine the blood pressure value of the user.

4. The method according to claim 3, wherein the method further comprises:

when the pressurization time period is less than the first time period, controlling, by the electronic device, the air pump to stop inflating and pressurizing the airbag, and prompting the user to tighten the wristband; and
when the pressurization time period is greater than the second time period, controlling, by the electronic device, the air pump to stop inflating and pressurizing the airbag, and prompting the user to loosen the wristband.

**5.** The method according to any one of claims 1-4, wherein the correcting, by the electronic device, a blood pressure measurement value based on the tightness, to determine a blood pressure value of the user comprises:

  obtaining, by the electronic device, pulse wave signal from the second pressure signal;
  determining the blood pressure measurement value of the user based on the pulse wave signal.

**6.** An electronic device, comprises:

  a wristband;
  an airbag;
  an air pump;
  a memory;
  a processor;
  a computer program that is stored in the memory and that can be run on the processor, wherein when executing the program, the processor is configured to:

    receive a first input;
    control the air pump to inflate and pressurize the airbag , after receiving the first input;
    obtain a first pressure signal, wherein the first pressure signal is a signal indicating that a pressure value of the airbag changes with time in a process in which the air pump starts to inflate and pressurize the airbag to a preset pressure value;
    determine, based on the first pressure signal, tightness of wearing the wristband by the user;
    correct, based on the tightness, a blood pressure measurement value determined based on a second pressure signal, to determine a blood pressure value of the user, wherein the second pressure signal is a signal indicating that the pressure value of the airbag changes with time after the pressure value of the airbag exceeds the preset pressure value wherein
    the processor is further configured to determine, based on the first pressure signal, a pressurization time period from a time point at which the airbag starts to be pressurized to a time point at which the pressure value of the airbag is equal to the preset pressure value, wherein the pressurization time period is used to indicate the tightness of wearing the wristband by the user; and
    the processor is further configured to correct the blood pressure measurement value based on the pressurization time period, to determine the blood pressure value of the user.

**Patentansprüche**

**1.** Blutdruck-Messverfahren, durchgeführt von einem elektronischen Gerät, wobei das elektronische Gerät ein Handgelenkband, ein Luftkissen und eine Luftpumpe umfasst und das elektronische Gerät an einem Handgelenk eines Benutzers getragen wird; und das Verfahren Folgendes umfasst:

  Empfangen (S1001), durch das elektronische Gerät, eines ersten Eingangs;
  Steuern der Luftpumpe, das Luftkissen aufzublasen und mit Druck zu beaufschlagen, nach dem Empfangen des ersten Eingangs;
  Erlangen (S1003), durch das elektronische Gerät, eines ersten Drucksignals, wobei das erste Drucksignal ein Signal ist, das angibt, dass ein Druckwert des Luftkissens in einem Prozess, in dem die Luftpumpe beginnt, das Luftkissen aufzublasen und mit Druck zu einem im Voraus eingestellten Druckwert zu beaufschlagen, sich mit der Zeit verändert;
  Bestimmen, durch das elektronische Gerät basierend auf dem ersten Drucksignal, einer Dichtheit des Tragens des Handgelenkbands durch den Benutzer; und
  Korrigieren, durch das elektronische Gerät basierend auf der Dichtheit, eines basierend auf einem zweiten Drucksignal bestimmten Blutdruck-Messwerts, um einen Blutdruckwert des Benutzers zu bestimmen, wobei das zweite Drucksignal ein Signal ist, das angibt, dass der Druckwert des Luftkissens sich mit der Zeit verändert, nachdem der Druckwert des Luftkissens den im Voraus eingestellten Druckwert überschreitet,
  wobei das Bestimmen, durch das elektronische Gerät basierend auf dem ersten Drucksignal, einer Dichtheit des Tragens des Handgelenkbands durch den Benutzer Folgendes umfasst:

    Bestimmen (S1004), durch das elektronische Gerät basierend auf dem ersten Drucksignal, einer Druckbeaufschlagung-Zeitperiode von einem Zeitpunkt, an dem das Luftkissen beginnt, mit Druck beaufschlagt zu werden, bis zu einem Zeitpunkt, an dem der Druckwert des Luftkissens gleich dem im Voraus eingestellten Druckwert ist, wobei die Druckbeaufschlagung-Zeitperiode verwendet wird, die Dichtheit des Tragens des Handgelenkbands durch den Benutzer anzugeben; und
    das Korrigieren, durch das elektronische Gerät, eines Blutdruck-Messwerts basierend auf der Dichtheit, um einen Blutdruckwert des Benutzers zu bestimmen, Folgendes umfasst:

Korrigieren (S1005), durch das elektronische Gerät, des Blutdruck-Messwerts basierend auf der Druckbeaufschlagung-Zeitperiode, um den Blutdruckwert des Benutzers zu bestimmen.

**2.** Verfahren nach Anspruch 1, wobei das Korrigieren (S1005), durch das elektronische Gerät, des Blutdruck-Messwerts basierend auf der Druckbeaufschlagung-Zeitperiode, um den Blutdruckwert des Benutzers zu bestimmen, Folgendes umfasst:

Bestimmen, durch das elektronische Gerät, eines Kompensationswerts basierend auf einer Abbildungsbeziehung, wobei die Abbildungsbeziehung eine Abbildungsbeziehung zwischen der Druckbeaufschlagung-Zeitperiode und dem Kompensationswert umfasst; und Korrigieren, durch das elektronische Gerät, des Blutdruck-Messwerts unter Verwendung des Kompensationswerts, um den Blutdruckwert des Benutzers zu bestimmen.

**3.** Verfahren nach Anspruch 1 oder 2, wobei das Korrigieren (S1005), durch das elektronische Gerät, des Blutdruck-Messwerts basierend auf der Druckbeaufschlagung-Zeitperiode, um den Blutdruckwert des Benutzers zu bestimmen, Folgendes umfasst: wenn die Druckbeaufschlagung-Zeitperiode größer als eine erste Zeitperiode und kleiner als eine zweite Zeitperiode ist, Korrigieren, durch das elektronische Gerät, des Blutdruck-Messwerts basierend auf der Druckbeaufschlagung-Zeitperiode, um den Blutdruckwert des Benutzers zu bestimmen.

**4.** Verfahren nach Anspruch 3, wobei das Verfahren ferner Folgendes umfasst:

wenn die Druckbeaufschlagung-Zeitperiode kleiner als die erste Zeitperiode ist, Steuern, durch das elektronische Gerät, der Luftpumpe, zu stoppen, das Luftkissen aufzublasen und mit Druck zu beaufschlagen, und Auffordern des Benutzers, das Handgelenkband zu straffen; und wenn die Druckbeaufschlagung-Zeitperiode größer als die zweite Zeitperiode ist, Steuern, durch das elektronische Gerät, der Luftpumpe, zu stoppen, das Luftkissen aufzublasen und mit Druck zu beaufschlagen, und Auffordern des Benutzers, das Handgelenkband zu lockern.

**5.** Verfahren nach einem der Ansprüche 1-4, wobei das Korrigieren, durch das elektronische Gerät, eines Blutdruck-Messwerts basierend auf der Dichtheit, um einen Blutdruckwert des Benutzers zu bestimmen, Folgendes umfasst:

Erlangen, durch das elektronische Gerät, eines Pulswellensignals von dem zweiten Drucksignal; Bestimmen des Blutdruck-Messwerts des Benutzers basierend auf dem Pulswellensignal.

**6.** Elektronisches Gerät, umfassend:

ein Handgelenkband; ein Luftkissen; eine Luftpumpe; einen Speicher; einen Prozessor; ein Computerprogramm, das in dem Speicher gespeichert ist und das auf dem Prozessor ausgeführt werden kann, wobei der Prozessor, wenn er das Programm ausführt, konfiguriert ist zum:

Empfangen eines ersten Eingangs; Steuern der Luftpumpe, das Luftkissen aufzublasen und mit Druck zu beaufschlagen, nach dem Empfangen des ersten Eingangs; Erlangen eines ersten Drucksignals, wobei das erste Drucksignal ein Signal ist, das angibt, dass ein Druckwert des Luftkissens in einem Prozess, in dem die Luftpumpe beginnt, das Luftkissen aufzublasen und zu einem im Voraus eingestellten Druckwert mit Druck zu beaufschlagen, sich mit der Zeit verändert; Bestimmen, basierend auf dem ersten Drucksignal, einer Dichtheit des Tragens des Handgelenkbands durch den Benutzer; Korrigieren, basierend auf der Dichtheit, eines basierend auf einem zweiten Drucksignal bestimmten Blutdruck-Messwerts, um einen Blutdruckwert des Benutzers zu bestimmen, wobei das zweite Drucksignal ein Signal ist, das angibt, dass der Druckwert des Luftkissens sich mit der Zeit verändert, nachdem der Druckwert des Luftkissens den im Voraus eingestellten Druckwert überschreitet, wobei der Prozessor ferner konfiguriert ist zum Bestimmen, basierend auf dem ersten Drucksignal, einer Druckbeaufschlagung-Zeitperiode von einem Zeitpunkt, an dem das Luftkissen beginnt, mit Druck beaufschlagt zu werden, bis zu einem Zeitpunkt, an dem der Druckwert des Luftkissens gleich dem im Voraus eingestellten Druckwert ist, wobei die Druckbeaufschlagung-Zeitperiode verwendet wird, die Dichtheit des Tragens des Handgelenkbands durch den Benutzer anzugeben; und der Prozessor ferner konfiguriert ist zum Korrigieren des Blutdruck-Messwerts basierend auf der Druckbeaufschlagung-Zeitperiode, um den Blutdruckwert des Benutzers zu bestimmen.

## Revendications

1. Procédé de mesure de la pression artérielle, mis en œuvre par un dispositif électronique, le dispositif électronique comprenant un bracelet, une vessie, et une pompe à air, et le dispositif électronique étant porté sur un poignet d'un utilisateur ; et le procédé comprenant :

   la réception (S1001), par le dispositif électronique, d'une première entrée ;
   la commande de la pompe à air pour gonfler et mettre sous pression la vessie, après réception de la première entrée ;
   l'obtention (S1003), par le dispositif électronique, d'un premier signal de pression, le premier signal de pression étant un signal indiquant qu'une valeur de pression de la vessie change au fil du temps dans un processus au cours duquel la pompe à air commence à gonfler et mettre sous pression la vessie jusqu'à une valeur de pression prédéfinie ;
   la détermination, par le dispositif électronique sur la base du premier signal de pression, de la tension de port du bracelet par l'utilisateur ; et
   la correction, par le dispositif électronique sur la base de la tension, d'une valeur de mesure de pression artérielle déterminée sur la base d'un second signal de pression, pour déterminer une valeur de pression artérielle de l'utilisateur, le second signal de pression étant un signal indiquant que la valeur de pression de la vessie change au fil du temps après que la valeur de pression de la vessie dépasse la valeur de pression prédéfinie,
   la détermination, par le dispositif électronique sur la base du premier signal de pression, de la tension de port du bracelet par l'utilisateur comprenant :

   la détermination (S1004), par le dispositif électronique sur la base du premier signal de pression, d'une période de temps de pressurisation depuis un point temporel au niveau duquel la vessie commence à être pressurisée jusqu'à un point temporel au niveau duquel la valeur de pression de la vessie est égale à la valeur de pression prédéfinie, la période de temps de pressurisation étant utilisée pour indiquer la tension de port du bracelet par l'utilisateur ; et
   la correction, par le dispositif électronique, d'une valeur de mesure de pression artérielle sur la base de la tension, pour déterminer une valeur de pression artérielle de l'utilisateur comprenant :
   la correction (S1005), par le dispositif électronique, de la valeur de mesure de pression artérielle sur la base de la période de temps de pressurisation, pour déterminer la valeur de pression artérielle de l'utilisateur.

2. Procédé selon la revendication 1, la correction (S1005), par le dispositif électronique, de la valeur de mesure de pression artérielle sur la base de la période de temps de pressurisation, pour déterminer la valeur de pression artérielle de l'utilisateur comprenant :

   la détermination, par le dispositif électronique, d'une valeur de compensation sur la base d'une relation de correspondance, la relation de correspondance comprenant une relation de correspondance entre la période de temps de pressurisation et la valeur de compensation ; et
   la correction, par le dispositif électronique, de la valeur de mesure de pression artérielle en utilisant la valeur de compensation, pour déterminer la valeur de pression artérielle de l'utilisateur.

3. Procédé selon la revendication 1 ou 2, la correction (S1005), par le dispositif électronique, de la valeur de mesure de pression artérielle sur la base de la période de temps de pressurisation, pour déterminer la valeur de pression artérielle de l'utilisateur comprenant :
   lorsque la période de temps de pressurisation est supérieure à une première période de temps et inférieure à une seconde période de temps, la correction, par le dispositif électronique, de la valeur de mesure de pression artérielle sur la base de la période de temps de pressurisation, pour déterminer la valeur de pression artérielle de l'utilisateur.

4. Procédé selon la revendication 3, le procédé comprenant en outre :

   lorsque la période de temps de pressurisation est inférieure à la première période de temps, la commande, par le dispositif électronique, de la pompe à air pour arrêter de gonfler et mettre sous pression la vessie, et l'invitation à l'utilisateur de serrer le bracelet ; et
   lorsque la période de temps de pressurisation est supérieure à la seconde période de temps, la commande, par le dispositif électronique, de la pompe à air d'arrêter de gonfler et mettre sous pression la vessie, et l'invitation à l'utilisateur de desserrer le bracelet.

5. Procédé selon l'une quelconque des revendications 1 à 4, la correction, par le dispositif électronique, d'une valeur de mesure de pression artérielle sur la base de la tension, pour déterminer une valeur de pression artérielle de l'utilisateur comprenant :

    l'obtention, par le dispositif électronique, d'un signal d'onde de pouls à partir du second signal de pression ;
    la détermination de la valeur de mesure de pression artérielle de l'utilisateur sur la base du signal d'onde de pouls.

6. Dispositif électronique, comprenant :

    un bracelet ;
    une vessie ;
    une pompe à air ;
    une mémoire ;
    un processeur ;
    un programme informatique qui est stocké dans la mémoire et qui peut être exécuté sur le processeur, lors de l'exécution du programme, le processeur étant configuré pour :

        recevoir une première entrée ;
        commander la pompe à air pour gonfler et mettre sous pression la vessie, après réception de la première entrée ;
        obtenir un premier signal de pression, le premier signal de pression étant un signal indiquant qu'une valeur de pression de la vessie change au fil du temps dans un processus au cours duquel la pompe à air commence à gonfler et mettre sous pression la vessie jusqu'à une valeur de pression prédéfinie ;
        déterminer, sur la base du premier signal de pression, la tension de port du bracelet par l'utilisateur ;
        corriger, sur la base de la tension, une valeur de mesure de pression artérielle déterminée sur la base d'un second signal de pression, pour déterminer une valeur de pression artérielle de l'utilisateur, le second signal de pression étant un signal indiquant que la valeur de pression de la vessie change au fil du temps après que la valeur de pression de la vessie dépasse la valeur de pression prédéfinie
        le processeur étant en outre configuré pour déterminer, sur la base du premier signal de pression, une période de temps de pressurisation depuis un point temporel au niveau duquel la vessie commence à être pressurisée jusqu'à un point temporel au niveau duquel la valeur de pression de la vessie est égale à la valeur de pression prédéfinie, la période de temps de pressurisation étant utilisée pour indiquer la tension de port du bracelet par l'utilisateur ; et
le processeur étant en outre configuré pour corriger la valeur de mesure de pression artérielle sur la base de la période de temps de pressurisation, pour déterminer la valeur de pression artérielle de l'utilisateur.

FIG. 1

Electronic device 200

Storage unit
202

Communications
line
206

Pressure
sensor
205

Communications
line
206

MCU
201

Communications
line
206

Wristband
207

Air pump
203

Communications
line
206

Airbag
204

Communications line
206

FIG. 2

Wristband 320

Watch body 310

Display 312

Physical
buttons 311

Airbag 330

FIG. 3(a)                    FIG. 3(b)

Original signal in an initial
pressurization phase

Pressure value (mmHg)

Time (s)

FIG. 4(a)                    FIG. 4(b)                    FIG. 4(c)

S501

A blood pressure watch receives a first input, where an air pump inflates and pressurizes an airbag

S502

The blood pressure watch obtains a pressure value of the airbag in a process of controlling the air pump to inflate and pressurize the airbag

S503

The blood pressure watch obtains a first pressure signal, where the first pressure signal is a signal indicating that the pressure value of the airbag changes with time in a process in which the air pump starts to inflate and pressurize the airbag until the pressure value of the airbag is equal to a preset pressure value

S504

The blood pressure watch determines a pressurization slope of the first pressure signal, where the pressurization slope is used to indicate tightness of wearing a wristband by a user

S505

The blood pressure watch corrects, based on the pressurization slope of the first pressure signal, a blood pressure measurement value determined based on a second pressure signal, to determine a blood pressure value of the user, where the second pressure signal is a signal indicating that the pressure value of the airbag changes with time after the pressure value of the airbag exceeds the preset pressure value

FIG. 5

Physical button
311-1

FIG. 6(a)

Display 312

Blood pressure
measurement

FIG. 6(b)

FIG. 7

FIG. 8(a)

Display 312

The wristband is worn too loosely. Please tighten the wristband and restart blood pressure measurement

FIG. 8(b)

Display 312

The wristband is worn too tightly. Please loosen the wristband and restart blood pressure measurement

FIG. 9

EP 4 023 149 B1

S1001 — A blood pressure watch receives a first input, where an air pump inflates and pressurizes an airbag

S1002 — The blood pressure watch obtains a pressure value of the airbag in a process of controlling the air pump to inflate and pressurize the airbag

S1003 — The blood pressure watch obtains a first pressure signal, where the first pressure signal is a signal indicating that the pressure value of the airbag changes with time in a process in which the air pump starts to inflate and pressurize the airbag until the pressure value of the airbag is equal to a preset pressure value

S1004 — The blood pressure watch determines a pressurization time period from a time point at which the airbag starts to be pressurized to a time point at which the pressure value of the airbag is equal to the preset pressure value, where the pressurization time period is used to indicate tightness of wearing a wristband by a user

S1005 — The blood pressure watch corrects, based on the pressurization time period, a blood pressure measurement value determined based on a second pressure signal, to determine a blood pressure value of the user, where the second pressure signal is a signal indicating that the pressure value of the airbag changes with time after the pressure value of the airbag exceeds the preset pressure value

FIG. 10

FIG. 11

EP 4 023 149 B1

Electronic device

Obtaining unit
1202

Control unit
1201

Prompt unit
1204

Determining unit
1203

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2011295130 A1
- CN 109984736 A
- US 6336901 B1